(19) Europäisches Patentamt
European Patent Office
Office européen des brevets

(11) EP 4 717 249 A1

(12) **EUROPEAN PATENT APPLICATION**

(43) Date of publication:
**01.04.2026 Bulletin 2026/14**

(21) Application number: **25202411.2**

(22) Date of filing: **16.09.2025**

(51) International Patent Classification (IPC):
***A61K 6/17*** *(2020.01)* ***A61K 6/62*** *(2020.01)*
***A61K 6/76*** *(2020.01)* ***A61K 6/887*** *(2020.01)*
***A61K 6/20*** *(2020.01)* ***A61K 6/30*** *(2020.01)*

(52) Cooperative Patent Classification (CPC):
(C-Sets available)
**A61K 6/887; A61K 6/17; A61K 6/20; A61K 6/30; A61K 6/62; A61K 6/76** (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **25.09.2024 JP 2024166521**

(71) Applicant: **Shofu Inc.**
**Kyoto-shi, Kyoto 605-0983 (JP)**

(72) Inventors:
- **SHINTANI, Naoto**
  **Kyoto-shi, Kyoto, 605-0983 (JP)**
- **MIYATA, Shunsuke**
  **Kyoto-shi, Kyoto, 605-0983 (JP)**
- **ANDO, Hitoshi**
  **Kyoto-shi, Kyoto, 605-0983 (JP)**
- **NAKAGAMI, Kazuki**
  **Kyoto-shi, Kyoto, 605-0983 (JP)**

(74) Representative: **Eisenführ Speiser Patentanwälte Rechtsanwälte PartGmbB**
**Gollierstraße 4**
**80339 München (DE)**

(54) **DENTAL COMPOSITION**

(57) The problem to be solved by the present invention is to provide a filler that can be used to fabricate a dental composition with stable paste properties even after long-term storage, and to provide the dental composition.

A dental composition including an (A) polymerizable monomer, a (B) polymerization initiator, and a (C) filler, characterized in that the (C) filler includes a (c2-1) filler having an average primary particle size of greater than 100 nm, surface-treated with a silane coupling agent, and when the (c2-1) filler having an average primary particle size of greater than 100 nm, surface-treated with a silane coupling agent, undergoes a thermogravimetric analysis under conditions of a heating rate of 10°C/min from 30°C to 550°C in a nitrogen atmosphere, a weight loss rate at 250°C is 50% or less of a weight loss rate at 550°C.

EP 4 717 249 A1

(52) Cooperative Patent Classification (CPC): (Cont.)

C-Sets
**A61K 6/76, C08K 9/06;**
**A61K 6/887, C08L 33/10**

## Description

BACKGROUND ART

Technical Field

The present invention relates to a dental composition.

Description of the Related Art

In the field of dentistry, dental compositions are used for treatment of an oral cavity, and are practically used in dental adhesives, dental composite resins, dental core construction materials, dental resin cement, dental coating materials, dental pit and fissure sealants, dental manicure materials, dental adhesives for fixing mobile teeth, dental glass ionomer cement, dental hard resins, dental milling materials, materials for dental 3D printers, materials for orthodontics, and the like.

In clinical dentistry, in order to restore aesthetics and functionality of teeth that have been lost due to caries, fractures, and the like, treatment has been carried out employing a direct method for repairing them using an dental adhesive and a composite resin, and an indirect method for repairing a dental prosthesis composed of ceramic or a dental hard resin with dental resin cement. Moreover, dental adhesives for attaching a dental composite resin to various dental materials and natural teeth, dental adhesives for fixing mobile teeth for fixation of mobile teeth, dental coating materials for protecting a vital tooth with hyperesthesia or after formation from external irritation and secondary caries, dental pit and fissure sealants for preventing caries by filling deep fissures in a molar with the sealant, dental manicure materials for temporarily restoring aesthetics by masking discoloration of teeth, dental abutment construction materials for forming an abutment tooth when a dental crown portion of a tooth has collapsed due to caries, are also used. In recent years, new composite materials have been developed, such as dental milling materials for creating a dental prosthesis using CAD/CAM processing, dental materials for 3D printers for creating a dental prosthesis using 3D printers, and orthodontic materials used to treat malocclusion and the like, a variety of which are used in treatment.

The materials as described above are mixed with a resin matrix composed of several types of polymerizable monomers, various fillers such as an inorganic filler and an organic-inorganic composite filler, and a polymerization initiator, according to their purpose to prepare homogeneous paste. An example of some of the materials includes a dental filling composite resin which is used by filling it into a tooth in a state of unhardened paste, imparting an anatomical shape of a natural tooth with dental appliances such as dental instruments, irradiating it with light using a dental light irradiator or the like to harden it. The light for use, irradiated from the light irradiator is generally a light source with an output of approximately 100 to 3500 $mW/cm^2$ in the wavelength range of approximately 360 to 500 nm. Dental resin cement is, on the other hand, used in a case in which a dental prosthesis adheres to a cavity or abutment tooth, and is hardened by irradiation with light after the dental prosthesis has been attached to the cavity or abutment tooth.

The dental composition is configured of a polymerizable monomer, a polymerization initiator, a filler, and the like. A filler treated with a surface treating agent such as a silane coupling agent is used, and examples of a type of filler include an inorganic filler, an organic filler, and an organic-inorganic composite filler. Treatment with the surface treating agent is carried out for the purpose of improving compatibility between the filler and the polymerizable monomer and improving the physical properties of a dental composition fabricated, such as bending strength, compressive strength, and dischargeability from a syringe.

Patent literatures (WO 2014/083842, WO 2016/152659, and WO 2020/031444) describe a technique for adjusting a consistency of a dental composition using a filler treated with a silane coupling agent having a specific structure.

SUMMARY

When a filler having a long time elapsed after surface treatment with a silane coupling agent is used, the dental composition fabricated has resulted in problems of its reduced consistency and a decrease in physical properties such as syringe dischargeability, compared to a dental composition fabricated using a filler that has been surface-treated immediately after surface treatment. Therefore, an object of the present invention is to provide a filler capable of realizing fabrication of dental composition wherein paste has stable properties even after long-term storage, and to provide the dental composition.

The present inventors have found as a result of diligent investigations that the aforementioned problems can be solved by using a specific dental composition.

The present disclosure provides the following items.

(Item 1)

A dental composition, comprising an (A) polymerizable monomer, a (B) polymerization initiator, and a (C) filler, wherein the (C) filler comprises a (c2-1) filler having an average primary particle size of greater than 100 nm, surface-treated with a silane coupling agent, and when the (c2-1) filler having an average primary particle size of greater than 100 nm, surface-treated with a silane coupling agent, undergoes a thermogravimetric analysis under conditions of a heating rate of 10°C/min from 30°C to 550°C in a nitrogen atmosphere, a weight loss rate at 250°C is 50% or less of a weight loss rate at 550°C.

(Item 2)

The dental composition according to item 1, wherein the silane coupling agent has a structure represented by formula (1):

[Formula (1)]

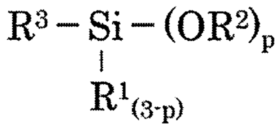

wherein $R^1$ and $R^2$ are C1 to C4 alkyl groups optionally having the same or different groups from each other, $R^3$ is a (meth) acryloyl group having a C6 to C15 alkyl group optionally having one or more groups selected from -O-, -S-, -NH-, -C(O)-O-, -O-C(O)-, -O-C(O)-NH-, and -NH-C(O)-O- groups, and
p is 2 or 3.

(Item 3)

The dental composition according to item 1 or 2, wherein the (c2-1) filler having an average primary particle size of greater than 100 nm, surface-treated with a silane coupling agent, is a mixture of a surface treatment liquid comprising the silane coupling agent, a catalyst and a solvent, and a (c1) untreated filler.

(Item 4)

The dental composition according to item 3, wherein the surface treatment liquid is a mixed solution aged at a temperature below a boiling point of the solvent.

(Item 5)

The dental composition according to any one of items 1 to 4, wherein a consistency retention ratio of a dental composition compounded with the (c2-1) filler having an average primary particle size of greater than 100 nm, surface-treated with a silane coupling agent after storage at 50°C for 90 days after surface treatment, is 85% or more of a consistency of a dental composition compounded with the (c2-1) filler having an average primary particle size of greater than 100 nm, surface-treated with a silane coupling agent immediately after surface treatment.

(Item 6)

The dental composition according to any one of items 1 to 5, wherein an increased ratio in extrusion load of the dental composition compounded with the (c2-1) filler having an average primary particle size of greater than 100 nm, surface-treated with a silane coupling agent after storage at 50°C for 90 days after surface treatment, is 10% or less of an extrusion load of a dental composition compounded with the (c2-1) filler having an average primary particle size of greater than 100 nm, surface-treated with a silane coupling agent immediately after surface treatment.

(Item 7)

The dental composition according to any one of items 1 to 6, comprising 0.5 to 10 parts by mass of the (B) polymerization initiator and 100 to 900 parts by mass of the (c2-1) filler having an average primary particle size of greater

than 100 nm, surface-treated with a silane coupling agent, relative to a total of 100 parts by mass of the (A) polymerizable monomer,

wherein a ratio of content of the (C) untreated filler to content of a silane coupling agent ([(c1) untreated filler/silane coupling agent], mass ratio, in terms of solid content) in the (c2-1) filler having an average primary particle size of greater than 100 nm, surface-treated with a silane coupling agent that is a mixture of the surface treatment liquid comprising the silane coupling agent, a catalyst and a solvent, and the (c1) untreated filler, is 100/0.5 to 20.

Advantageous Effects of Invention

The present inventors have revealed that using a filler having a weight loss rate at 250°C that is 50% or less of a weight loss rate at 550°C upon thermogravimetric analysis under conditions of a heating rate of 10°C/min from 30°C to 550°C in a nitrogen atmosphere, enables providing a filler that gives the same dental consistency of a dental composition fabricated, even in case of having a long time elapsed after surface treatment of the filler, as in case of using the filler immediately after surface treatment, and does not deteriorate a property such as syringe dischargeability, i.e., to provide a filler that exhibits excellent storage stability, to solve the problems. Moreover, the dental composition compounded with such a filler is characterized by exhibiting high bending strength.

BRIEF DESCRIPTION OF THE DRAWINGS

FIG. 1 shows a thermogravimetric analysis curve of a filler treated with a surface treatment liquid in which hydrolysis and dehydration (condensation) reactions of a silane coupling agent have not sufficiently proceeded; and
FIG. 2 shows a thermogravimetric analysis curve of a filler treated with a surface treatment liquid in which hydrolysis and dehydration (condensation) reactions of a silane coupling agent have sufficiently proceeded.

DETAILED DESCRIPTION OF THE PREFERRED EMBODIMENT(S)

Each component of the dental composition of the present invention will be described in detail below.

[(A) Polymerizable monomer]

The (A) polymerizable monomer contained in the dental composition of the present invention can be limitlessly used as long as it is of any publicly known polymerizable monomer. In the polymerizable monomer as used herein, its polymerizable group is preferably a group that exhibits radical polymerizability and more specifically, from the viewpoint of facilitation of radical polymerization, the polymerizable group is more preferably a (meth)acrylic group and/or a (meth) acrylamide group. It is to be noted that the "(meth)acrylic" used herein means acrylic and/or " methacrylic, the "(meth) acryloyl" means acryloyl and/or methacryloyl, the "(meth)acrylate" means acrylate and/or methacrylate, and the "(meth) acrylamide" means acrylamide and/or methacrylamide. A polymerizable monomer having a substituent at the $\alpha$-position of a (meth)acrylic group and/or a (meth)acrylamide group can also be preferably used. Examples of the (A) polymerizable monomer include one or more selected from a polymerizable monomer having one radically polymerizable group, no acidic group, no alkoxysilyl group, and no sulfur atom, a polymerizable monomer having two radically polymerizable groups, no acidic group, no alkoxysilyl group, and no sulfur atom, a polymerizable monomer having three radically polymerizable groups, no acidic group, no alkoxysilyl group, and no sulfur atom, a polymerizable monomer having an acidic group, a polymerizable monomer having an alkoxysilyl group, a polymerizable monomer having an alkoxysilyl group as well as a urethane group and/or an ether group, a polymerizable monomer having a sulfur atom, a polymerizable monomer having a urethane group and/or an ether group, and a silane coupling agent.

Specific examples of the polymerizable monomer having one radically polymerizable group, no acidic group, no alkoxysilyl group, and no sulfur atom include one or more selected from 2-hydroxyethyl(meth)acrylate, 3-hydroxypropyl(meth)acrylate, 4-hydroxybutyl(meth)acrylate, 2-hydroxypropyl(meth)acrylate, 2-hydroxybutyl(meth)acrylate, 6-hydroxyhexyl(meth)acrylate, 10-hydroxydecyl(meth)acrylate, propylene glycol mono(meth)acrylate, glycerol mono(meth) acrylate, erythritol mono(meth)acrylate, N-methylol(meth)acrylamide, N-hydroxyethyl(meth)acrylamide, N,N-(dihydroxyethyl) (meth)acrylamide, methyl(meth)acrylate, ethyl(meth)acrylate, propyl(meth)acrylate, isopropyl(meth)acrylate, butyl(meth)acrylate, isobutyl(meth)acrylate, benzyl(meth)acrylate, lauryl(meth)acrylate, 2,3-dibromopropyl(meth)acrylate, 3-(meth)acryloyloxypropyltrimethoxysilane, 11-(meth)acryloyloxyundecyltrimethoxysilane, or (meth)acrylamide.

Specific examples of the polymerizable monomer having two radically polymerizable groups, no acidic group, no alkoxysilyl group, and no sulfur atom include one or more selected from 2,2-bis((meth)acryloyloxyphenyl)propane, 2,2-bis [4-(3-(meth)acryloyloxy)-2-hydroxypropoxyphenyl]propane (commonly known as "Bis-GMA"), 2,2-bis(4-(meth)acryloyloxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytetraethoxyphenyl)propane, 2,2-bis(4-(meth)acryloyloxypentaethoxyphenyl) propane, 2,2-bis(4-(meth)acryloyloxydipropoxyphenyl)propane, 2-(4-(meth)acryloyloxydiethoxyphenyl)-2-(4-(meth)acryloyloxydiethoxyphenyl)propane, 2-(4-(meth)acryloyloxydiethoxyphenyl)-2-(4-(meth)acryloyloxyditriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, 2,2-bis(4-(meth)acryloyloxyisopropoxyphenyl)propane, 1,4-bis(2-(meth)acryloyloxyethyl)pyromellitate, glycerol di(meth)acrylate, 1-(acryloyloxy)-3-(methacryloyloxy)-2-propanol, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, polyethylene glycol di(meth)acrylate, 1,3-butanediol di(meth)acrylate, 1,5-pentanediol di(meth)acrylate, 1,6-hexanediol di(meth)acrylate, 1,10-decanediol di(meth)acrylate, 1,2-bis(3-methacryloyloxy-2-hydroxypropoxy) ethane, 2,2,4-trimethylhexamethylenebis(2-carbamoyloxyethyl)dimethacrylate (commonly known as "UDMA"), or 1,2-bis(3-methacryloyloxy-2-hydroxypropoxy)ethane.

Specific examples of the polymerizable monomer having three radically polymerizable groups, no acidic group, no alkoxysilyl group, and no sulfur atom include one or more selected from trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol penta(meth)acrylate, N,N-(2,2,4-trimethylhexamethylene)bis[2-(aminocarboxy) propane-1,3-diol]tetramethacrylate, or 1,7-diacryloyloxy-2,2,6,6-tetraacryloyloxymethyl-4-oxyheptane.

Examples of the polymerizable monomer having an alkoxysilyl group include (meth)acrylic-based and (meth) acrylamide-based compounds, having one alkoxysilyl group in the molecule, and (meth)acrylic-based and (meth) acrylamide-based compounds, having multiple alkoxysilyl groups in the molecule. Specific examples of the polymerizable monomer having an alkoxysilyl group include one or more selected from 2-(meth)acryloxyethyltrimethoxysilane, 3-(meth) acryloxypropyltrimethoxysilane, 3-(meth)acryloxypropyltriethoxysilane, 3-(meth)acryloxypropylmethyldimethoxysilane, 4-(meth)acryloxybutyltrimethoxysilane, 5-(meth)acryloxypentyltrimethoxysilane, 6-(meth)acryloxyhexyltrimethoxysilane, 7-(meth)acryloxyheptyltrimethoxysilane, 8-(meth)acryloxyoctyltrimethoxysilane, 9-(meth)acryloxynonyltrimethoxysilane, 10-(meth)acryloxydecyltrimethoxysilane, or 11-(meth)acryloxyundecyltrimethoxysilane. Examples of the polymerizable monomer having an alkoxysilyl group and a urethane group and/or an ether group include one or more selected from 3,3-dimethoxy-8,37-dioxo-2,9,36-trioxa-7,38-diaza-3-silatetracontan-40-yl(meth)acrylate, 2-((3,3-dimethoxy-8-oxo-2,9,18-trioxa-7-aza-3-silanonadecane-19-oyl)amino)-2-methylpropane-1,3-diyldi(meth)acrylate, 3,3-dimethoxy-8,19-dioxo-2,9,18-trioxa-7,20-diaza-3-siladocosane-22-yl(meth)acrylate, 3,3-dimethoxy-8,22-dioxo-2,9,12,15,18,21-hexaoxa-7,23-diaza-3-silapentacosane-25-yl(meth)acrylate, 3,3-dimethoxy-8,22-dioxo-2,9,12,15,18,21,26-heptaoxa-7,23-diaza-3-silaoctacosane-28-yl(meth)acrylate, 3,3-dimethoxy-8,19-dioxo-2,9,12,15,18-pentaoxa-7,20-diaza-3-siladocosane-22-yl(meth)acrylate, 3,3-dimethoxy-8,19-dioxo-2,9,12,15,18,23-hexaoxa-7,20-diaza-3-silapentacosane-25-yl(meth)acrylate, 2-((3,3-dimethoxy-8-oxo-2,9,12,15,18-pentaoxa-7-aza-3-silanonadecane-19-oyl)amino)-2-methylpropane-1,3-diyldi(meth)acrylate, 4,4-diethoxy-17-oxo-3,16,21-trioxa-18-aza-4-silatricosane-23-yl(meth)acrylate, 4,4-diethoxy-17-oxo-3,16,21,24-tetraoxa-18-aza-4-silahexacosane-26-yl(meth)acrylate, 4,4-diethoxy-13-oxo-3,12,17-trioxa-14-aza-4-silanonadecane-19-yl(meth)acrylate, 4,4-diethoxy-17-oxo-3,16-dioxa-18-aza-4-silaicosane-20-yl(meth)acrylate, or 2-methyl-2-((11-(triethoxysilyl)undecyloxy)carbonylamino)propane-1,3-diyldi(meth)acrylate.

The dental composition of the present invention may contain a polymerizable monomer having a sulfur atom as the (A) polymerizable monomer in order to impart adhesiveness to a precious metal. The polymerizable monomer having a sulfur atom may be any publicly known compound without any limitations as long as it is a polymerizable monomer having one or more sulfur atoms and polymerizable groups. Specifically, it refers to a compound having a partial structure such as SH, S-S, C=S, C-S-C, or P=S, or a compound resulting from tautomerization. Specific examples thereof include one or more selected from 10-methacryloxydecyl-6,8-dithiooctanate, 6-methacryloxyhexyl-6,8-dithiooctanate, 6-methacryloyloxyhexyl 2-thiouracil-5-carboxylate, 2-(11-methacryloyloxyundecylthio)-5-mercapto-1,3,4-thiadiazole, or 10-(meth)acryloyloxydecyl dihydrogen thiophosphate.

In addition to these polymerizable monomers, an oligomer or a prepolymer having at least one or more polymerizable groups in a molecule can be used without any limitations. Also, there is no problem even though such an oligomer or a prepolymer may have a substituent such as a fluoro group in the same molecule. The above-described polymerizable monomers can be used singly or in combination with a plurality thereof.

The dental composition of the present invention may contain a silane coupling agent as the (A) polymerizable monomer in order to impart adhesiveness to a glass ceramic. Any publicly known silane coupling agent may be used without limitations, but one or more selected from 3-methacryloxypropyltrimethoxysilane, 8-methacryloxyoctyltrimethoxysilane, 11-methacryloxyundecyltrimethoxysilane, or the like are preferable. From the viewpoint of imparting adhesiveness, the silane coupling agent is compounded in an amount of preferably 1 part by mass or more and more preferably 5 parts by mass or more and less than 20 parts by mass, relative to 100 parts by mass of the total amount of polymerizable monomer in the composition. The silane coupling agent as the polymerizable monomer is compounded with an aim to impart adhesiveness to resin materials containing a glass ceramic and a filler composed of a glass ceramic, and the like,

and it is therefore compounded separately from a surface treating agent for the filler.

The dental composition of the present invention may contain a polymerizable monomer having a sulfur atom as the (A) polymerizable monomer in order to impart adhesiveness to a precious metal. The amount of polymerizable monomer having a sulfur atom compounded is preferably 0.01 parts by mass or more and more preferably 0.1 parts by mass or more and less than 20 parts by mass, relative to the total of 100 parts by mass of the polymerizable monomer contained in the dental composition, from the viewpoint of imparting adhesiveness.

The polymerizable monomer contained in the dental composition of the present invention has no problem in containing a polymerizable monomer having a cationically polymerizable functional group, however, it is preferable to contain a polymerizable monomer having a radically polymerizable functional group singly. In case of containing the polymerizable monomer having a cationically polymerizable functional group, storage stability may decrease.

Examples of the polymerizable monomer having a cationically polymerizable functional group include one or more selected from a vinyl ether compound, an epoxy compound, an oxetane compound, a cyclic ether compound, or a cyclic carbonate compound.

The dental composition of the present invention may contain an (A1) polymerizable monomer having an acidic group. The polymerizable monomer having an acidic group can be used without any limitations as long as it is a polymerizable monomer having one or more polymerizable groups and having one or more acidic groups selected from a phosphoric acid group, a pyrophosphoric acid group, a thiophosphoric acid group, a phosphonic acid group, a sulfonic acid group, a carboxylic acid group, or the like. Including the polymerizable monomer having an acidic group enables adhesiveness to be imparted to teeth and prosthetic devices.

Specific examples of the polymerizable monomer having a phosphoric acid group include one or more selected from 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, bis[2-(meth)acryloyloxyethyl] hydrogen phosphate, bis[4-(meth)acryloyloxybutyl] hydrogen phosphate, bis[6-(meth)acryloyloxyhexyl] hydrogen phosphate, bis [8-(meth)acryloyloxyoctyl] hydrogen phosphate, bis[9-(meth)acryloyloxynonyl] hydrogen phosphate, bis[10-(meth)acryloyloxydecyl] hydrogen phosphate, 1,3-di(meth)acryloyloxypropyl dihydrogen phosphate, 2-(meth)acryloyloxyethylphenyl hydrogen phosphate, 2-(meth)acryloyloxyethyl-2-bromoethyl hydrogen phosphate, or bis[2-[meth]acryloyloxy-(1-hydroxy methyl)ethyl] hydrogen phosphate; acid chlorides, alkali metal salts, or ammonium salts thereof; or (meth) acrylamide compounds in which ester bonds of these compounds have been replaced with amide bonds.

Specific examples of the polymerizable monomer having a pyrophosphoric acid group include one or more selected from bis[2-(meth)acryloyloxyethyl] pyrophosphate, bis[4-(meth)acryloyloxybutyl] pyrophosphate, bis[6-(meth) acryloyloxyhexyl] pyrophosphate, bis[8-(meth)acryloyloxyoctyl] pyrophosphate, or bis[10-(meth)acryloyloxydecyl] pyrophosphate; acid chlorides, alkali metal salts, or ammonium salts thereof; or (meth)acrylamide compounds in which ester bonds of these compounds have been replaced with amide bonds.

Specific examples of the polymerizable monomer having a thiophosphoric acid group include one or more selected from 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphite, 6-(meth)acryloyloxyhexyl dihydrogen thiophosphate, 7-(meth)acryloyloxyheptyl dihydrogen thiophosphite, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate, 11-(meth)acryloyloxyundecyl dihydrogen thiophosphate, 12-(meth)acryloyloxydodecyl dihydrogen thiophosphate, 16-(meth)acryloyloxyhexadecyl dihydrogen thiophosphate, or 20-(meth)acryloyloxyicosyl dihydrogen thiophosphate; acid chlorides, alkali metal salts, or ammonium salts thereof; or (meth)acrylamide compounds in which ester bonds of these compounds have been replaced with amide bonds. It is to be noted that the polymerizable monomer having a thiophosphate group is also classified as a polymerizable monomer having a sulfur atom.

Specific examples of the polymerizable monomer having a phosphonic acid group include one or more selected from 2-(meth)acryloyloxyethyl phenyl phosphonate, 5-(meth)acryloyloxypentyl-3-phosphonopropionate, 6-(meth)acryloyloxyhexyl-3-phosphonopropionate, 10-(meth)acryloyloxydecyl-3-phosphonopropionate, 6-(meth)acryloyloxyhexyl-3-phosphonoacetate, 10-(meth)acryloyloxydecyl-3-phosphonoacetate; acid chlorides, alkali metal salts, or ammonium salts thereof; or (meth)acrylamide compounds in which ester bonds of these compounds have been replaced with amide bonds.

Specific examples of the polymerizable monomer having a sulfonic acid group include one or more selected from 2-(meth)acrylamide-2-methylpropanesulfonic acid or 2-sulfoethyl (meth)acrylate.

The polymerizable monomer having a carboxylic acid group is classified into a (meth)acrylic-based compound having one carboxyl group in the molecule, and a (meth)acrylic-based compound having a plurality of carboxyl groups in the molecule. Specific examples of the (meth)acrylic compound having one carboxyl group in the molecule include one or

more selected from (meth)acrylic acid, N-(meth)acryloylglycine, N-(meth)acryloylaspartic acid, O-(meth)acryloyltyrosine, N-(meth)acryloyltyrosine, N-(meth)acryloylphenylalanine, N-(meth)acryloyl-p-aminobenzoic acid, N-(meth)acryloyl-o-aminobenzoic acid, p-vinylbenzoic acid, 2-(meth)acryloyloxybenzoic acid, 3-(meth)acryloyloxybenzoic acid, 4-(meth)acryloyloxybenzoic acid, N-(meth)acryloyl-5-aminosalicylic acid, N-(meth)acryloyl-4-aminosalicylic acid, 2-(meth)acryloyloxyethyl hydrogen succinate, 2-(meth)acryloyloxyethyl hydrogen phthalate, or 2-(meth)acryloyloxyethyl hydrogen malate; acid halides thereof; or (meth)acrylamide compounds in which ester bonds of these compounds have been replaced with amide bonds. Specific examples of the (meth)acrylic-based compound having a plurality of carboxyl groups in the molecule include one or more selected from 6-(meth)acryloyloxyhexane-1,1-dicarboxylic acid, 9-(meth)acryloyloxynonane-1,1-dicarboxylic acid, 10-(meth)acryloyloxydecane-1,1-dicarboxylic acid, 11-(meth)acryloyloxyundecane-1,1-dicarboxylic acid, 12-(meth)acryloyloxydodecane-1,1-dicarboxylic acid, 13-(meth)acryloyloxytridecane-1,1-dicarboxylic acid, 4-(meth)acryloyloxyethyl trimellitate, 4-(meth)acryloyloxybutyl trimellitate, 4-(meth)acryloyloxyhexyl trimellitate, 4-(meth)acryloyloxydecyl trimellitate, or 2-(meth)acryloyloxyethyl-3'-(meth)acryloyloxy-2'-(3,4-dicarboxybenzoyloxy)propyl succinate; acid anhydrides and acid halides thereof; or (meth)acrylamide compounds in which ester bonds of these compounds have been replaced with amide bonds.

Preferred examples of the (A1) polymerizable monomer having an acidic group include one or more selected from (meth)acrylic acid, 10-methacryloyloxydecyl dihydrogen phosphate, 6-methacryloxyhexyl phosphonoacetate, 4-(meth)acryloyloxyethyl trimellitate, or acid anhydrides thereof. From the viewpoint of imparting adhesiveness, the amount of (A1) polymerizable monomer having an acidic group is preferably 1 part by mass or more and 20 parts by mass or less in 100 parts by mass of the (A) polymerizable monomer. The (A1) polymerizable monomer contained in an amount of 1 part by mass or more facilitates adhesiveness to be exhibited, and the (A1) polymerizable monomer contained in an amount of 20 parts by mass or less tends to result in having favorable storage stability and curing properties.

[(B) Polymerization initiator]

The polymerization initiator used in the dental composition of the present invention is not limited, and any publicly known radical generators can be used. A polymerization initiator is generally broadly classified into those that initiate polymerization by mixing immediately before use (chemical polymerization initiators), those that initiate polymerization by heating or warming (thermal polymerization initiators), and those that initiate polymerization by light irradiation (photopolymerization initiators), and the polymerization initiator can be selected therefrom according to their usage and purposes. Also, these polymerization initiators can be used singly or in combination with multiple initiators, regardless of a polymerization mode or a polymerization method. Furthermore, these polymerization initiators can be used without any problems even though they are subjected to secondary treatment such as encapsulation in microcapsules in order to achieve stabilization of polymerization, prolonged polymerization, and the like.

Examples of the chemical polymerization initiator include one or more selected from a redox-type polymerization initiation-based initiator or an organometallic polymerization initiator-based initiator, which reacts with oxygen or water to initiate polymerization. Specific examples of the redox-type polymerization initiation-based initiator include one or more selected from an organic peroxide, an amine compound, a sulfinate, or a borate compound. Examples of a preferred combination of the chemical polymerization initiators include a combination of an organic peroxide and an amine compound, a combination of an organic peroxide, an amine compound, and a sulfinate, or a combination of an organic peroxide, an amine compound, and a borate compound.

Specific examples of the organic peroxide include one or more selected from benzoyl peroxide, parachlorobenzoyl peroxide, 2,4-dichlorobenzoyl peroxide, acetyl peroxide, lauroyl peroxide, tertiary butyl peroxide, cumene hydroperoxide, 2,5-dihydroperoxide, methyl ethyl ketone peroxide, or tertiary butyl peroxybenzoate.

The amine compound is preferably a secondary or tertiary amine in which the amine group is bonded to an aryl group, and specific examples thereof include one or more selected from p-N,N-dimethyl-toluidine, N,N-dimethylaniline, N-β-hydroxyethyl-aniline, N,N-di(β-hydroxyethyl)-aniline, p-N,N-di(β-hydroxyethyl)-toluidine, N-methyl-aniline, or p-N-methyl-toluidine.

Specific examples of the sulfinate include one or more selected from sodium benzenesulfinate, lithium benzenesulfinate, or sodium p-toluenesulfinate. The sulfinate can also initiate polymerization by reacting with a polymerizable monomer having an acidic group.

Specific examples of the borate compound include one or more selected from a sodium salt, a lithium salt, a potassium salt, a magnesium salt, a tetrabutylammonium salt, a tetramethylammonium salt, and the like of trialkylphenylboron or trialkyl(p-fluorophenyl)boron (the alkyl group is a n-butyl group, a n-octyl group, a n-dodecyl group, and the like.). The borate compound can also initiate polymerization by reacting with a polymerizable monomer having an acidic group.

Specific examples of the organometallic polymerization initiator include one or more selected from organoboron compounds such as triphenylborane, tributylborane, and tributylborane partial oxide, or the Period 4 transition metal compounds.

Also, examples of the thermal polymerization initiator that works by heating or warming include one or more selected from an organic peroxide or an azo compound. Specific examples of the azo compound include one or more selected from azobisisobutyronitrile, methyl azobisisobutyrate, or azobiscyanovaleric acid.

Examples of the photopolymerization initiator include one or more selected from those composed of a photosensitizer, a combination of a photosensitizer and a polymerization accelerator, or a combination of a photosensitizer, a photoacid generator, and a polymerization accelerator. Of these, the photopolymerization initiator used in the dental composition of the present invention preferably includes a combination of a (B-1) photosensitizer, a (B-2) photoacid generator, and a (B-3) polymerization accelerator. Known compounds commonly used can be used therefore without any limitations.

[(B-1) Photosensitizer]

Specific examples of the (B-1) photosensitizer include one or more selected from α-diketones, benzoin alkyl ethers, thioxanthones, benzophenones, acylphosphine oxides, acylgermanium compounds, α-aminoacetophenones, ketals, or titanocenes.

Specific examples of the α-diketones include one or more selected from benzil, compounds of camphorquinones, or α-naphthyl. Specific examples of the compound of camphorquinones include one or more selected from camphorquinone, camphorquinone carboxylic acid, or camphorquinone sulfonic acid.

Specific examples of the benzoin alkyl ethers include one or more selected from benzoin, benzoin methyl ether, or benzoin ethyl ether.

Specific examples of thioxanthones include one or more selected from thioxanthone, 2-chlorothioxanthone, or 2-methylthioxanthone.

Specific examples of the benzophenones include one or more selected from benzophenone, p-chlorobenzophenone, or p-methoxybenzophenone.

Specific examples of acylphosphine oxides include one or more selected from bis(2,6-dimethoxybenzoyl) phenylphosphine oxide, bis(2,6-dimethoxybenzoyl) (2,4,4-trimethylpentyl)phosphine oxide, bis(2,6-dimethoxybenzoyl)-n-butylphosphine oxide, bis(2,4,6-trimethylbenzoyl)phenylphosphine oxide, or 2,4,6-trimethylbenzoyldiphenylphosphine oxide.

Specific examples of the acylgermanium compound include one or more selected from bisbenzoyldiethylgermanium, bisbenzoyldimethylgermanium, or bisbenzoyldibutylgermanium.

Specific examples of the α-aminoacetophenones include one or more selected from 2-benzyl-dimethylamino-1-(4-morpholinophenyl)-1-butanone, or 2-benzyl-diethylamino-1-(4-morpholinophenyl)-1-propanone.

Specific examples of the ketals include one or more selected from benzyl dimethyl ketal, benzyl diethyl ketal, or benzyl (2-methoxyethyl ketal).

Specific examples of the titanocenes include one or more selected from bis(cyclopentadienyl)-bis[2,6-difluoro-3-(1-pyrrolyl)phenyl]-titanium, bis(cyclopentadienyl)-bis(pentanefluorophenyl)-titanium, or bis(cyclopentadienyl)-bis(2,3,5,6-tetrafluoro-4-disiloxyphenyl)-titanium.

The (B-1) photosensitizer can be appropriately selected depending on a wavelength, intensity, and a light exposure time of light used for polymerization, and a type and the compounding amount of another component to be combined for use. The photosensitizer can be also used singly or in combination of two or more thereof. Among them, a compound of α-diketones having a maximum absorption wavelength in the visible light region is preferably used, with the compound of camphorquinones being more preferred, one or more selected from camphorquinone, camphorquinone carboxylic acid, camphorquinone sulfonic acid, or the like, being still more preferred, and camphorquinone being particularly preferred in terms of facilitation of availability.

The dental composition of the present invention may contain α-diketone compounds singly as the (B-1) photosensitizer.

[(B-2) Photoacid generator]

The (B-2) photoacid generator to be used in the dental composition of the present invention, which is a publicly known compound can be used without any limitations. Specifically, examples thereof include one or more selected from a triazine compound, an iodonium salt-based compound, a sulfonium salt-based compound, or a sulfonic acid ester compound. Among these, one or more selected from the triazine compound or iodonium salt-based compound are preferred because of their high polymerizability when used in combination with a sensitizer. The iodonium salt-based compound is more preferred. The iodonium salt-based compound is prone to be sensitized by a photosensitizer that has absorption in the visible light region.

Specific examples of the triazine compound include one or more selected from 2,4,6-tris(trichloromethyl)-s-triazine, 2,4,6-tris(tribromomethyl)-s-triazine, or 2-methyl-4,6-bis(trichloromethyl)-s-triazine. Among these, 2,4,6-tris(trichloromethyl)-s-triazine is preferred.

Any publicly known iodonium salt-based compounds can be used. Among the iodonium salt-based compounds, an aryl iodonium salt is preferred because of their high stability. Also, the aryl group preferably has a substituent in order to improve fat solubility. Specifically, linear alkyl groups such as methyl, propyl, octyl, decyl, undecyl, dodecyl, and tridecyl groups, branched alkyl groups such as isopropyl, isobutyl, sec-butyl, tert-butyl, isopentyl, neopentyl, tert-pentyl, and isohexyl, or a functional group in which one or more H atoms in these hydrocarbon groups have been substituted with F, a perfluoroalkyl group, a halogen, and the like, are suitable for substituents.

Specific examples of the iodonium salt-based compound include one or more selected from diphenyliodonium hexafluorophosphate, p-cumenyl(p-tolyl)iodonium tris(pentafluoroethyl)trifluorophosphate, bis(4-tert-butylphenyl)iodonium tris(pentafluoropropyl)trifluorophosphate, bis(4-tert-butylphenyl)iodonium tetrakispentafluorophenylborate, bis [4-(tert-butyl)phenyl]iodonium tetra(nonafluoro-tert-butoxy)aluminate, bis(4-tert-butylphenyl)iodonium tetrakispentafluorophenylgallate, or bis(4-tert-butylphenyl)iodonium nonafluorobutanesulfonate.

The photoacid generator that can be used in the dental composition of the present invention is not limited to the photoacid generators shown as specific examples, and two or more types thereof can be combined for use.

[(B-3) Polymerization accelerator]

The (B-3) polymerization accelerator to be used in the dental composition of the present invention is not limited as long as it has polymerization accelerating ability, and any publicly known polymerization accelerators generally used in the dental field can be used without any limitations. Examples of the (B-3) polymerization accelerator include one or more selected from primary to tertiary amine compounds, a phosphine compound, an organometallic compound, the Period 4 transition metal compounds, a thiourea derivative, sulfinic acid and a salt thereof, a borate compound, a sulfur-containing reductive inorganic compound, a nitrogen-containing reductive inorganic compound, a barbituric acid derivative, a triazine compound, or a halogen compound. Specific examples of the primary to tertiary amine compounds include one or more selected from an aromatic amine compound or an aliphatic amine compound. When the phrase "(numerical value) to (numerical value)" is used herein, it is meant to include each numerical value.

The aromatic amine compound refers to a compound in which one or more H atoms of ammonia ($NH_3$) are substituted with an aromatic ring. A compound in which one H atom of $NH_3$ is substituted with an aromatic ring can be classified as an aromatic primary amine compound, a compound in which one H atom of $NH_3$ is substituted with an aromatic ring and another H atom is substituted with an aromatic ring or an alkyl group can be classified as an aromatic secondary amine compound, and a compound in which one H atom of $NH_3$ is substituted with an aromatic ring and the other two different H atoms are substituted with an aromatic ring or an alkyl group can be classified as an aromatic tertiary amine compound.

Specific examples of the aromatic primary amine compound include aniline, specific examples of the aromatic secondary amine compound include a N-protected amino acid (ester), and specific examples of the aromatic tertiary amine compound include one or more selected from N,N-dimethylaniline, N,N-diethylaniline, p-N,N-diethyl-toluidine, p-dimethylaminobenzoic acid ethyl ester, or N,N-di-n-butylaniline. Specific examples of the N-protected amino acid (ester) include one or more selected from N-phenylbenzylamine, N-benzyl-p-anisidine, or N-benzyl-o-phenetidine. Among these, p-dimethylaminobenzoic acid ethyl ester is preferred.

The aliphatic amine compound refers to a compound in which one or more H atoms of ammonia ($NH_3$) are substituted with an alkyl group. The alkyl group is classified as follows: $CH_3$- or $CH_2$- is a primary alkyl group, a group in which one H atom of -$CH_2$- may be substituted with a substituent is a secondary alkyl group, and a group in which two H atoms of -$CH_2$- are substituted with an alkyl substituent is a tertiary alkyl group. The aliphatic amine is classified as follows: A compound in which one H atom of $NH_3$ is substituted with an alkyl group is a primary aliphatic amine compound, a compound in which two H atoms of $NH_3$ are substituted with an alkyl group is a secondary aliphatic amine compound, and a compound in which three H atoms of $NH_3$ are substituted with an alkyl group is a tertiary aliphatic amine compound.

Specific examples of the aliphatic primary amine compound include one or more selected from benzhydrylamine, triphenylmethylamine, or glycine. Specific examples of the aliphatic secondary amine compound include one or more selected from dibenzylamine, N-benzyl-1-phenylethylamine, or bis(1-phenylethyl)amine. Specific examples of the aliphatic tertiary amine compound include one or more selected from tributylamine, tripropylamine, triethylamine, N-methyldiethanolamine, triethanolamine, tribenzylamine, dibenzylglycine ethyl ester, N,N-dimethylaminoethyl methacrylate, or N-diethylaminoethyl methacrylate.

The phosphine compound refers to a compound in which the P atom is substituted with three organic groups, and an aromatic phosphine compound refers to a compound in which the P atom is substituted with a phenyl group optionally having one or more substituents. Specific examples of the phosphine compound include one or more selected from trimethylphosphine, tributylphosphine, triphenylphosphine, 4-(phenylphosphino)benzoic acid, tri(o-tolyl)phosphine, tri(m-tolyl)phosphine, tri(p-tolyl)phosphine, or trihexylphosphine. Among these, preferred are one of more selected from triphenylphosphine, 4-(phenylphosphino)benzoic acid, tri(o-tolyl)phosphine, tri(m-tolyl)phosphine, tri(p-tolyl)phosphine,

and the like.

Specific examples of the organometallic compound include organometallic compounds containing one or more selected from titanium (Ti), vanadium (V), manganese (Mn), iron (Fe), copper (Cu), tin (Sn), zinc (Zn), zirconium (Zr), or the like, and preferred is organometallic compounds containing one or more selected from tin (Sn), vanadium (V), copper (Cu), or the like. Specific examples of the organometallic compound containing tin (Sn) include one or more selected from dibutyl-tin-diacetate, dibutyl-tin-dimaleate, dioctyl-tin-dilaurate, dibutyl-tin-dilaurate, or dioctyl-tin-dimaleate. Specific examples of the organometallic compound containing vanadium (V) include one or more selected from vanadium acetylacetonate, divanadium tetroxide, or vanadyl acetylacetonate. Specific examples of the organometallic compound containing copper (Cu) include one or more selected from copper acetylacetonate, copper naphthenate, or copper octylate.

Among these, preferred are one or more selected from a trivalent or tetravalent vanadium compound or a divalent copper compound, with a trivalent or tetravalent vanadium compound having higher polymerization accelerating ability being more preferred, and a tetravalent vanadium compound being the most preferred. These Period 4 transition metal compounds may be used in combination with multiple types thereof, if necessary. The amount of transition metal compound compounded is preferably 0.0001 to 1 part by mass relative to 100 parts by mass of the total amount of (A) polymerizable monomer. When the amount thereof is 0.0001 part by mass or more, a sufficient polymerization accelerating effect tends to result, and the amount thereof of 1 part by mass or less makes it unlikely to cause discoloration and gelation of the dental composition, which therefore tends to have favorable storage stability.

Specific examples of the thiourea derivative include one or more selected from dimethylthiourea, diethylthiourea, (2-pyridyl)thiourea, N-acetylthiourea, N-benzoylthiourea, or tetramethylthiourea. Among these, preferred are one or more selected from (2-pyridyl) thiourea, N-acetylthiourea, or N-benzoylthiourea. Multiple types of these thiourea derivatives may be combined for use, if necessary. The amount of thiourea derivative compounded is preferably 0.1 to 5 parts by mass relative to 100 parts by mass of the total amount of (A) polymerizable monomer. The amount of 0.1 part by mass or more tends to result in satisfactory polymerization accelerating ability, and the amount of 5 parts by mass or less tends to result in favorable storage stability.

Specific examples of sulfinic acid and a salt thereof include one or more selected from p-toluenesulfinic acid, sodium p-toluenesulfinate, sodium benzenesulfinate, sodium 2,4,6-triisopropylbenzenesulfinate, or potassium p-toluenesulfinate. Among these, particularly preferred are one or more selected from sodium benzenesulfinate, sodium p-toluenesulfinate, sodium 2,4,6-triisopropylbenzenesulfinate, or the like.

Examples of the borate compound include one or more selected from a borate compound having one aryl group in one molecule, a borate compound having two aryl groups in one molecule, a borate compound having three aryl groups in one molecule, or a borate compound having four aryl groups in one molecule. Specific examples of the borate compound having one aryl group in one molecule include trialkylphenyl boron, trialkyl(p-chlorophenyl) boron, or trialkyl (p-fluorophenyl) boron. Specific examples of the borate compound having two aryl groups in one molecule include one or more selected from dialkyldiphenyl boron, dialkyldi(p-chlorophenyl) boron, or dialkyldi(p-fluorophenyl) boron. Specific examples of the borate compound having three aryl groups in one molecule include one or more selected from monoalkyltriphenyl boron, monoalkyltri(p-chlorophenyl) boron, or monoalkyltri(p-fluorophenyl) boron. Specific examples of the borate compound having four aryl groups in one molecule include one or more selected from tetraphenyl boron, tetrakis(p-chlorophenyl) boron, or tetrakis(p-fluorophenyl) boron.

Among these borate compounds, it is more preferable to use a borate compound having three or four aryl groups in one molecule from the viewpoint of storage stability. It is also possible to use these borate compounds in admixture of one or more thereof.

Examples of the reductive inorganic compound containing sulfur include one or more selected from a sulfite, a bisulfite, a pyrosulfite, a thiosulfate, a thionate, or dithionite. Specific examples thereof include one or more selected from sodium sulfite, potassium sulfite, calcium sulfite, ammonium sulfite, sodium hydrogen sulfite, potassium hydrogen sulfite, 3-mercaptopropyltrimethoxysilane, 2-mercaptobenzoxazole, decanethiol, or thiobenzoic acid.

An example of the reductive inorganic compound containing nitrogen includes a nitrite. Specific examples thereof include one or more selected from sodium nitrite, potassium nitrite, calcium nitrite, or ammonium nitrite.

Specific examples of the barbituric acid derivative include one or more selected from barbituric acid, 1,3-dimethylbarbituric acid, 1,3-diphenylbarbituric acid, 1-benzyl-5-phenylbarbituric acid, or salts of thiobarbituric acids (preferably alkali metal or alkaline earth metal). Specific examples of the salts of these barbituric acids include one or more selected from sodium 5-butylbarbiturate, sodium 1,3,5-trimethylbarbiturate, or sodium 1-cyclohexyl-5-ethylbarbiturate.

Specific examples of the halogen compound include one or more selected from dilauryl dimethyl ammonium chloride, lauryl dimethyl benzyl ammonium chloride, benzyl trimethyl ammonium chloride, tetramethyl ammonium chloride, benzyl dimethyl cetyl ammonium chloride, or dilauryl dimethyl ammonium bromide.

There is no problem even though these (B-1) photosensitizer, (B-2) photoacid generator, and (B-3) polymerization accelerator, which are polymerization initiators, are subjected to secondary treatment such as micro pulverization,

adsorption onto a support, or encapsulation in a microcapsule, if necessary. Furthermore, these various types of photopolymerization initiators can be used singly or in combination of two or more thereof, regardless of polymerization modes or polymerization methods.

The amount of (B) polymerization initiator is preferably 0.5 to 10 parts by mass relative to 100 parts by mass of the total amount of (A) polymerizable monomer. The amount of 0.5 parts by mass or more leads to favorable curing properties, and the amount of 10 parts by mass or less allows excellent environmental light stability to be exhibited.

The (B) polymerization initiator preferably contains a combination of the (B-1) photosensitizer, the (B-2) photoacid generator, and the (B-3) polymerization accelerator. In this case, the (B) polymerization initiator preferably contains a diaryliodonium salt compound as the (B-2) photoacid generator. It also preferably contains a tertiary amine compound as the (B-3) polymerization accelerator. Containing the diaryliodonium salt compound as the (B-2) photoacid generator and the tertiary amine compound as the (B-3) polymerization accelerator enables excellent photocuring properties to be imparted.

More preferably, it is preferable that the (B-3) polymerization accelerator is substantially free of an aromatic amine compound. The phrase "substantially free" means that the compound is not intentionally compounded. For example, in a case in which an aromatic amine is contained in impurities and the like of a component contained in the dental composition, or in a case in which the aromatic amine is contained only in a trace amount of less than 0.1 parts by mass relative to 100 parts by mass of the total amount of (A) polymerizable monomer contained in the dental composition, these cases correspond to "substantially free" as long as they do not affect properties of the dental composition. In case of using the aromatic amine compound as the polymerization accelerator, it tends to exhibit higher polymerization accelerating ability than when an aliphatic amine compound is used, however, there is a problem that solar stability is significantly reduced. Therefore, in case of using the aromatic amine compound, it is necessary to compound a large amount of ultraviolet-ray absorber to ensure the solar stability, as a result of which fluorescence and mechanical properties may be deteriorated. In a case in which the dental composition of the present invention contains an amine compound as the (B-3) polymerization accelerator, the amine compound is preferably an aliphatic amine compound.

[(C)Filler]

Specific examples of the (C) filler include one or more selected from an inorganic filler, an organic filler, an organic-inorganic composite filler, or ion-sustained release glass. The dental composition of the present invention may use the exemplified fillers singly or in combination of two or more thereof.

Specific examples of the inorganic filler, which is not limited in terms of its chemical composition, include one or more selected from silicon dioxide, alumina, titania, silica-titania, silica-titania-barium oxide, silica-zirconia, silica-alumina, lanthanum glass, borosilicate glass, soda glass, barium glass, strontium glass, glass ceramic, aluminosilicate glass, barium boroaluminosilicate glass, strontium boroaluminosilicate glass, fluoroaluminosilicate glass, calcium fluoroaluminosilicate glass, strontium fluoroaluminosilicate glass, barium fluoroaluminosilicate glass, or strontium calcium fluoroaluminosilicate glass. In particular, one or more selected from barium fluoroaluminosilicate glass, strontium fluoroaluminosilicate glass, fluoroaluminosilicate glass, and the like, which are used in dental glass ionomer cement, resin-reinforced glass ionomer cement, resin cement, and the like, can also be preferably used. The fluoroaluminosilicate glass referred to herein has a basic backbone of silicon oxide and aluminum oxide and contains an alkali metal for introduction of non-crosslinked oxygen. The fluoroaluminosilicate glass further contains an alkaline earth metal containing strontium as a modifying and coordinating ion, and fluorine. The fluoroaluminosilicate glass is also a composition in which a lanthanoid series element is incorporated into a backbone to impart further X-ray opacity. The lanthanoid series element is also incorporated into the composition as a modifying and coordinating ion depending on a composition range.

Specific examples of the organic filler include one or more polymers selected from polymethyl methacrylate, polyethyl methacrylate, a methyl methacrylate-ethyl methacrylate copolymer, an ethyl methacrylate-butyl methacrylate copolymer, a methyl methacrylate-trimethylolpropane methacrylate copolymer, polyvinyl chloride, polystyrene, chlorinated polyethylene, nylon, polysulfone, polyethersulfone, or polycarbonate.

Examples of the organic-inorganic composite filler include a filler, a surface of which has been coated by polymerizing a polymerizable monomer, a filler in which it has been mixed with a polymerizable monomer, and the mixture has been then polymerized and pulverized to an appropriate particle size, a filler in which it has been dispersed in a polymerizable monomer, and the mixture has been then emulsion-polymerized or suspension-polymerized, a filler in which it has been dispersed in a polymerizable monomer and a solvent, and the mixture has been then spray-dried then polymerized, and a filler in which it has been dispersed in a solvent, and the mixture has been then spray-dried and then impregnated with a polymerizable monomer followed by polymerization, but the fillers are in no way limited thereto.

The ion-sustained release glass is characterized by releasing at least one or more ions selected from a fluoride ion, a strontium ion, a borate ion, an aluminum ion, or a silicate ion. Multiple ions are preferably released simultaneously.

As the ion-sustained release glass used herein, any ion-sustained release glass can also be limitlessly used as long as it contains one or more glass backbone-forming elements that form a glass backbone and one or more glass

modifying elements that modify the glass backbone. These pieces of ion-sustained release glass can be used singly or in combination with a plurality of pieces of the ion-sustained release glass. Also, in the present invention, a glass amphoteric element that plays the role of both of a glass backbone-forming element or a glass modifying element depending on a glass composition is included in the category of glass backbone-forming elements. Specific examples of the glass skeleton-forming element contained in the ion-sustained release glass include one or more selected from silica, aluminum, boron, or phosphorus. Specific examples of the glass modifying element include one or more selected from a halogen element, an alkali metal element, or an alkaline earth metal element. Specific examples of the halogen group element include one or more selected from fluorine, bromine, or iodine. Specific examples of the alkali metal group element include one or more selected from sodium or lithium. Specific examples of the alkaline earth metal group element include one or more selected from calcium or strontium. Among these, it is preferable to include as the glass backbone-forming element, one or more selected from silica, aluminum, or boron, and to include as the glass modifying element, one or more selected from fluorine, sodium, or strontium. Specific examples of the ion-sustained release glass containing these elements include silica glass, fluoroaluminosilicate glass, fluoroborosilicate glass, or fluoroaluminoborosilicate glass, which contain strontium and/or sodium. Furthermore, from the viewpoint of sustained-releasing a fluoride ion, a strontium ion, a borate ion, and an aluminum ion, more preferred is fluoroaluminoborosilicate glass containing strontium. Specific examples of glass composition ranges include $SiO_2$: 15 to 35% by mass, $Al_2O_3$: 15 to 30% by mass, $B_2O_3$: 5 to 20% by mass, SrO: 20 to 45% by mass, F: 5 to 15% by mass, and $Na_2O$: 0 to 10% by mass. This glass composition can be confirmed by using one or more of instrumental analyses selected from elemental analysis, Raman spectrum method, or fluorescent X-ray analysis, but there is no problem as long as an actual measurement value matches these composition ranges in any of the analysis methods.

A method for producing these ion-sustained release glasses is not limited, and they can be produced by a production method such as a melting method or a sol-gel method. Among them, a production method employing a melting method using a melting furnace is preferable from the viewpoint of facilitation of glass composition design including selection of raw materials. The ion-sustained release glass used herein has an amorphous structure, but has no problem despite partially including a crystalline structure, and further has no problem in spite of being a mixture of glass having an amorphous structure and glass having a crystalline structure. Whether the glass structure is amorphous or not can be confirmed adopting X-ray diffraction analysis or using analytical equipment such as a transmission electron microscope. Among these glasses, the ion-sustained release glass releases various ions due to equilibrium relation with ion concentrations thereof in an external environment, so that the ion-sustained release glass to be used in the present invention preferably has a homogeneous amorphous structure.

Furthermore, in order to enhance ion releasability from the ion-sustained release glass, a glass surface is preferably functionalized by surface treatment to improve the ion releasability, which is a preferred aspect. Specific examples of a surface treating agent to be used for the surface treatment include one or more selected from a surfactant, a fatty acid, an organic acid, an inorganic acid, a monomer, a polymer, various coupling agents (excluding a silane coupling agent), a silane compound, a metal alkoxide compound, or a partial condensate thereof. Among these surface treating agents, it is preferable to perform composite surface treatment using an acidic polymer and a silane compound.

The composite surface treatment is a method for coating a surface of the ion-sustained release glass with a silane compound followed by surface treatment with an acidic polymer, and will be specifically described below. A silane compound represented by formula (2) is mixed into an aqueous dispersion containing ion-sustained release glass that has undergone micro pulverization to a desired average particle size (D50) by pulverization or the like, and is then hydrolyzed or partially hydrolyzed in the system to form a silanol compound, and then condensed to form a polysiloxane, with which a surface of the ion-sustained release glass is then coated, to form polysiloxane-coated ion-sustained release glass.

[Formula (2)]

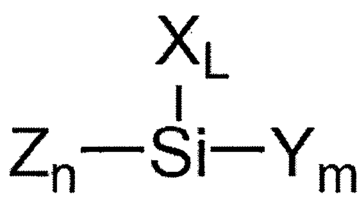

wherein in formula (2),

Z is $RO^-$,
X is a halogen,
Y is $OH^-$,

R is an organic group of C8 or less,
n, m, and L are integers of 0 to 4, and
n + m + L = 4.

Specific examples of the silane compound represented by formula (2) include one or more selected from tetramethoxysilane, tetraethoxysilane, tetrapropoxysilane, tetraallyloxysilane, tetrabutoxysilane, tetrakis(2-ethylhexyloxy)silane, trimethoxychlorosilane, triethoxychlorosilane, triisopropoxychlorosilane, trimethoxyhydroxysilane, diethoxydichlorosilane, tetraphenoxysilane, tetrachlorosilane, or silicon hydroxide (silicon oxide hydrate), and it is more preferably one or more selected from tetramethoxysilane or tetraethoxysilane.

Also, the polysiloxane-coated filler is more preferably a low condensate of the silane compound represented by formula (2). For example, it is a low condensate of a silane compound obtained by partially hydrolyzing and condensing tetramethoxysilane and tetraethoxysilane. These compounds may be used singly or in combination thereof. An organosilane compound can also be added as part of the silane compound represented by formula (2) upon polysiloxane treatment.

The polysiloxane-coated ion-sustained release glass obtained in the previous step can be subjected to acidic polymer treatment whereby the glass is reacted with an acidic polymer to obtain ion-sustained release glass. For the acidic polymer treatment, any equipment commonly used in the industry can be used as long as it is a dry fluidized mixer. Specific examples of the dry fluidized mixer include a Henschel mixer, a super mixer, or a high-speed mixer. The reaction of the acidic polymer with the ion-sustained release glass on which a polysiloxane coated film has been formed, can be carried out by bringing the glass into contact with an acidic polymer solution by impregnation, spraying or the like. For example, the polysiloxane-coated ion-sustained release glass is fluidized in a dry atmosphere, and while it is maintained in the fluidized state, a solution containing an acidic polymer is dispersed from above and the mixture is only thoroughly stirred. In this case, a method for dispersing the solution containing the acidic polymer is not limited, however, a dropping or spraying method capable of uniformly dispersing the solution is more preferable. Also, the reaction is preferably carried out in the vicinity of room temperature, and as the temperature increases, the reaction between an acid-reactive element and the acidic polymer is accelerated, resulting in non-uniform formation of cement phase.

It is preferable to remove moisture from within the cement reaction phase by carrying out heat treatment after the reaction. Leaving moisture within the cement reaction phase is disadvantageous in terms of strength, but the filler of the present invention prevents mechanical strength from decreasing, resulting from a coated film formed using a coupling agent. A heat treatment method after the acidic polymer treatment is not limited, and can be carried out by any publicly known general method. A piece of equipment used for the heat treatment is preferably a box-type hot air dryer or the like, or a rotary heat treatment apparatus capable of uniform heating or the like. A heat treatment temperature is in the range of room temperature to 200°C and more preferably in the range of 40 to 150°C. When the temperature is lower than this range, an aqueous medium is not sufficiently removed, and when it is higher than this range, the organic phase of acidic polymer may be decomposed and discolored. Since a heat treatment time depends on the capacity of the dryer, and the like, it is of no problem as long as the aqueous medium can be sufficiently removed. After the heat treatment, the heat-treated product can be easily crushed by applying shear force or impact force, and a crushing method can be performed using the equipment used in the above reaction.

A solvent used for preparing an acidic polymer solution used in the reaction can be any solvent as long as the acidic polymer is dissolved, and examples thereof include one or more selected from water, ethanol, ethanol, or acetone. Among these, water is particularly preferred, which dissociates an acidic group of the acidic polymer and allows the group to react uniformly with a surface of a basic filler that is a core.

The weight-average molecular weight of the polymer dissolved in the acidic polymer solution is in the range of 2,000 to 50,000, preferably in the range of 5,000 to 40,000. In the case of treatment with an acidic polymer having a weight-average molecular weight of less than 2,000, an acidic polymer reaction phase is not formed in the polysiloxane-coated ion-sustained release glass, resulting in a tendency of low ion-releasability. In the case of treatment with an acidic polymer having a weight-average molecular weight of more than 50,000, on the other hand, the viscosity of the acidic polymer solution increases, making it difficult to treat the polysiloxane-coated ion-sustained release glass uniformly. Also, an acidic polymer concentration in 100 parts by mass of the acidic polymer solution is preferably in the range of 3 to 25 parts by mass and more preferably in the range of 8 to 20 parts by mass. The acidic polymer concentration less than 3 parts by mass results in the fragile acidic polymer reaction phase described above, and cannot provide an improved effect of ion-releasability. Also, the acidic polymer concentration exceeding 25 parts by mass makes a polysiloxane layer (porous) difficult to be uniformly diffused and cannot provide a homogeneous acidic polymer reaction phase, and a reaction also occurs immediately when the acidic polymer comes into contact with the polysiloxane-coated ion-sustained release glass, arising problems such as forming a firm aggregate resulting from the reaction. Also, the amount of acidic polymer solution added relative to the polysiloxane-coated ion-sustained release glass is preferably in the range of 6 to 40 parts by mass and more preferably 10 to 30 parts by mass. In terms of this amount added, the optimal amount of acidic polymer relative to the polysiloxane-coated ion-sustained release glass is 1 to 7 parts by mass, and the optimal amount of water is 10 to 25

parts by mass.

The acidic polymer that can be used in order to form an acidic polymer reaction phase on a surface of the polysiloxane-coated ion-sustained release glass by the above-described method can be used without any problems as long as it is a copolymer or homopolymer of a polymerizable monomer having one or more acidic groups selected from a phosphoric acid residue, a pyrophosphoric acid residue, a thiophosphoric acid residue, a carboxylic acid residue, a sulfonic acid group, or the like. Specific examples of these polymerizable monomers include one or more selected from acrylic acid, methacrylic acid, 2-chloroacrylic acid, 3-chloroacrylic acid, aconitic acid, mesaconic acid, maleic acid, itaconic acid, fumaric acid, glutaconic acid, citraconic acid, 4-(meth)acryloyloxyethoxycarbonylphthalic acid, 4-(meth)acryloyloxyethoxycarbonylphthalic anhydride, 5-(meth)acryloylaminopentylcarboxylic acid, 11-(meth)acryloyloxy-1,1-undecanedicarboxylic acid, 2-(meth)acryloyloxyethyl dihydrogen phosphate, 10-(meth)acryloyloxydecyl dihydrogen phosphate, 20-(meth)acryloyloxyeicosyl dihydrogen phosphate, 1,3-di(meth)acryloyloxypropyl-2-dihydrogen phosphate, 2-(meth)acryloyloxyethyl phenyl phosphate, 2-(meth)acryloyloxyethyl-2'-bromoethyl phosphate, (meth)acryloyloxyethyl phenyl phosphonate, di(2-(meth)acryloyloxyethyl) pyrophosphate, 2-(meth)acryloyloxyethyl dihydrogen dithiophosphate, or 10-(meth)acryloyloxydecyl dihydrogen thiophosphate. Among the polymers (co)polymerized using these polymerizable monomers, it is preferable to use a homopolymer or copolymer of an $\alpha$-$\beta$ unsaturated carboxylic acid, which undergoes a relatively slow acid-base reaction with an acid reactive element contained in the polysiloxane-coated ion-sustained release glass, and specific examples thereof include an acrylic acid polymer, an acrylic acid-maleic acid copolymer, and an acrylic acid-itaconic acid copolymer.

A shape of the (C) filler is not limited, and a filler such as a spherical, needle-like, platy, crushed, or scaly shape, can be used.

An average particle size of the (C) filler, excluding the following (c2-1) and (c2-2) fillers, is preferably in the range of 0.01 μm to 50 μm, more preferably 0.01 μm to 30 μm, still more preferably 0.05 μm to 20 μm, and particularly preferably 0.05 μm to 10 μm. The average particle size used herein can be an average particle size calculated based on the volume-based particle size distribution measured, for example, by a laser diffraction particle size distribution measurement apparatus and the like, and can be measured, for example, by a laser diffraction particle size measurement apparatus (Microtrac MT3300EXII: manufactured by NIKKISO CO., LTD.). In addition thereto, a primary particle size can be measured by dynamic light scattering particle size measurement or by using an electron microscope photograph in a case where a primary particle is strongly aggregated to form a secondary particle.

[(c1) Untreated filler]

The dental composition of the present invention may contain a (c1) untreated filler that is not surface-treated with a silane coupling agent.

[(c2) Surface-treated filler]

The dental composition of the present invention may contain a (c2) surface-treated filler in which the (c1) untreated filler has been surface-treated with a silane coupling agent.

[(c2-1) filler having an average primary particle size of greater than 100 nm, surface-treated with a silane coupling agent]

Among the (c2) surface-treated fillers, the dental composition of the present invention contains the (c2-1) filler having an average primary particle size of greater than 100 nm, surface-treated with a silane coupling agent. The (c2-1) filler having an average primary particle size of greater than 100 nm, surface-treated with a silane coupling agent is contained in an amount of preferably 100 to 900 parts by mass, more preferably 100 to 800 parts by mass, particularly preferably 100 to 700 parts by mass, and even more particularly preferably 100 to 600 parts by mass, relative to 100 parts by mass of

(A) polymerizable monomer in total.

The average particle size of the (c2-1) filler having an average primary particle size of greater than 100 nm, surface-treated with a silane coupling agent, is preferably in the range of more than 100 nm and 50 μm or less, more preferably more than 100 nm and 30 μm or less, still more preferably more than 100 nm and 20 μm or less, and particularly preferably more than 100 nm and 10 μm or less.

[Silane coupling agent]

The silane coupling agent for the filler (c2-1) having an average primary particle size of greater than 100 nm, surface-treated with a silane coupling agent, is preferably one or more selected from methyltrimethoxysilane, methyltriethoxysilane, methyltrichlorosilane, dimethyldichlorosilane, trimethylchlorosilane, vinyltrichlorosilane, vinyltriethoxysilane, vinyltris(2-methoxyethoxy)silane, 3-methacryloyloxypropyltrimethoxysilane, 3-methacryloyloxypropyltriethoxysilane, 3-chloropropyltrimethoxysilane, 3-glycidoxypropyltrimethoxysilane, 8-methacryloyloxyoctyltrimethoxysilane, 10-methacryloyloxydecyltrimethoxysilane, 11-methacryloyloxyundecyltrimethoxysilane, 4,4-diethoxy-17-oxo-3,16-dioxa-18-aza-4-silaicosane-20-yl methacrylate, 4,4-diethoxy-17-oxo-3,16,21-trioxa-18-aza-4-silatricosane-23-yl methacrylate, hexamethyldisilazane, or a compound of the following formula (1):
[Formula (1)]

[Formula 4]

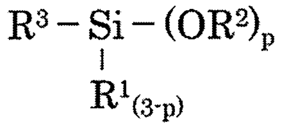

wherein in formula (1),

$R^1$ and $R^2$ are C1 to C4 alkyl groups and may be the same or different from each other,
$R^3$ is a (meth)acryloyl group having a C6 to C15 alkyl group, which may have one or more groups selected from - O-, -S-, -NH-, -C(O)-O-, -O-C(O)-, -O-C(O)-NH- or -NH-C(O)-O- group, and p is 2 or 3.

Examples of the C1 to C4 alkyl group in formula (1) include one or more selected from a methyl group, an ethyl group, a propyl group, an isopropyl group, a butyl group, an isobutyl group, a sec-butyl group, or a tert-butyl group.

The number of carbon atoms in an alkyl group of the silane coupling agent is preferably C6 or more. The number of carbon atoms of C6 or more results in favorable improved compatibility between the surface-treated filler and the polymerizable monomer, giving an effect of reducing an extrusion load of the dental composition fabricated and a high filling rate of the filler.

The ratio of content of the (c1) untreated filler to content of the silane coupling agent ([(c1) untreated filler/silane coupling agent], mass ratio, in terms of solid content) in the (C2-1) filler having an average primary particle size of greater than 100 nm, surface-treated with a silane coupling agent that is a mixture of a surface treatment liquid comprising a silane coupling agent, a catalyst and a solvent, and the (c1) untreated filler, is preferably 100/0.5 to 20.

For surface treatment of the filler, it is preferable to use a surface treatment liquid comprising a silane coupling agent and a solvent, and it is more preferable to use a surface treatment liquid comprising a silane coupling agent, a catalyst, and a solvent.

The catalyst is used for the purpose of accelerating hydrolysis or dehydration (condensation) of the silane coupling agent, and either an acid catalyst or a base catalyst may be used. Examples of the acid catalyst include one or more selected from inorganic acids (one or more selected from hydrochloric acid, nitric acid, phosphoric acid, sulfuric acid, boric acid, or the like) and organic acids (one or more selected from citric acid, acetic acid, lactic acid, tartaric acid, formic acid, propionic acid, or the like). Examples of the base catalyst include one or more selected from inorganic bases (one or more selected from ammonia water, sodium hydride, potassium hydride, calcium hydride, sodium hydroxide, potassium hydroxide, calcium hydroxide, sodium carbonate, potassium carbonate, calcium carbonate, sodium hydrogen carbonate, potassium hydrogen carbonate, or the like) and organic bases (one or more selected from tetramethylammonium hydroxide, tetraethylammonium hydroxide, sodium ethoxide, potassium t-butoxide, sodium amide, lithium diisopropylamide, sodium bis(trimethylsilyl)amide, pyridine, N,N-dimethyl-4-aminopyridine, trimethylamine, triethylamine, aniline, or the like). These catalysts can be used singly or in combination of two or more thereof. It is preferable to use an acid catalyst for the catalyst, and a pH of the acid catalyst is particularly preferably 4 or less. Examples of a method for adjusting a pH includes a method for adding an appropriate amount of acid catalyst to water to prepare an aqueous solution adjusted to a desired pH, and the like.

Examples of the solvent include one or more selected from water, ketones, ethers, esters, alcohols, aromatic hydrocarbons, aliphatic hydrocarbons, halogenated aliphatic hydrocarbons, or an aprotic polar solvent. Examples of the ketones include one or more selected from acetone, methyl ethyl ketone, methyl amyl ketone, acetylacetone, diethyl ketone, methyl amyl ketone, methyl isopropyl ketone, methyl isobutyl ketone, cyclopentanone, cyclohexanone, or isophorone. Examples of the ethers include one or more selected from diethyl ether, dioxane, tetrahydrofuran, tetrahydropyran, ethylene glycol dimethyl ether, tetraethylene glycol dimethyl ether, polyethylene glycol dimethyl ether, ethylene glycol monomethyl ether, ethylene glycol monoethyl ether, or propylene glycol monomethyl ether. Examples of the esters include one or more selected from methyl acetate, ethyl acetate, propyl acetate, butyl acetate, or methyl propionate. Examples of the alcohols include one or more selected from methanol, ethanol, isopropanol, n-propanol, isobutanol, n-butanol, pentanol, or acetone alcohol. Examples of the aromatic hydrocarbons include one or more selected from benzene, toluene, or xylene. Examples of the aliphatic hydrocarbons include one or more selected from hexane, heptane, octane, or cyclohexane. Examples of the halogenated aliphatic hydrocarbons include one or more selected from carbon tetrachloride, chloroform, trichloroethylene, methylene chloride, or 1,2-dichloroethane. Examples of the aprotic polar solvent include one or more selected from N,N-dimethylformamide, N-methylpyrrolidone, or dimethylsulfoxide. These solvents can be used singly or in combination of two or more thereof.

When the filler is subjected to surface treatment in the state of the silane coupling agent being not fully condensed, there is a possibility that an unreacted silanol group reacts on a filler surface during storage to cause a change in properties of the filler. Therefore, the surface treatment liquid is preferably aged in order to fully condense the silane coupling agent. The aging refers to treatment of condensing the silane coupling agent, and particularly refers to leaving liquid containing the silane coupling agent to stand at a specific temperature for a specific time. The lower limit of the aging time is preferably 2 hours or longer, more preferably 8 hours or longer, and still more preferably 24 hours or longer. The upper limit of the aging time is not limited, but is preferably 120 hours or shorter, more preferably 96 hours or shorter, and still more preferably 72 hours or shorter.

Upon aging, the surface treatment liquid comprising the silane coupling agent can be heated to further accelerate condensation. The higher the temperature upon heating, the more the condensation of the silane coupling agent is accelerated, so that the temperature upon heating is preferably as high as possible within the range of temperatures below the boiling point of the solvent to be used. The upper limit of the aging temperature is preferably 15°C or lower than the boiling point of the solvent to be used, more preferably 10°C or lower, and still more preferably 5°C or lower. The lower limit of the aging temperature is preferably 25°C or higher, more preferably 30°C or higher, and particularly preferably 40°C or higher.

The aging may be performed by a conventional method other than the aging in the surface treatment liquid. It is preferable to contain the silane coupling agent, which is a condensate of silanol groups obtained in such a manner, in the surface treatment liquid. In other words, it is preferable to use a surface treatment liquid comprising the condensed silane coupling agent, a catalyst, and a solvent.

The surface treatment method is not limited, and any publicly known methods can be employed without limitations, such as a method for spraying a surface treatment liquid while stirring a filler in powder form, a method for dispersing and mixing a filler in a surface treatment liquid, or a method for supplying a surface treatment liquid in a vapor or gas form to a filler surface. The amount treated with the surface treatment liquid in the filler is preferably 0.01 to 30 parts by mass and more preferably 0.5 to 20 parts by mass, relative to 100 parts by mass of the filler before treatment.

When the filler after completion of surface treatment step has been subjected to thermogravimetric analysis under conditions of a heating rate of 10°C/min from 30°C to 550°C in a nitrogen atmosphere, the ratio of the weight loss rate at 250°C to the weight loss rate at 550°C is preferably 50% or less, more preferably 40% or less, and particularly preferably 30% or less. In a case in which hydrolysis and dehydration (condensation) reactions of the silane coupling agent has not proceeded sufficiently at the time of surface treatment, as shown in FIG. 1, upon thermogravimetric analysis of the filler, a large weight loss at 250°C or lower is observed due to decomposition of a monomeric silane coupling agent. When the filler contains a large amount of monomeric silane coupling agent that has not undergone sufficient hydrolysis and dehydration (condensation) reactions, in the treated silane coupling agent, hydrolysis of unreacted alkoxy groups of the silane coupling agent and dehydration (condensation) of silanol groups proceed on a filler surface due to moisture in the air and the like, causing a change in wettability of the filler over time. As a result, when comparing the case where the filler is used immediately after surface treatment with the case where the filler is used with an elapsed time after the surface treatment, there occur changes in a consistency of paste, dischargeable powerfulness from a syringe, and the like, making it difficult to obtain a curable composition exhibiting stable physical properties. When hydrolysis and dehydration (condensation) reactions of the silane coupling agent sufficiently proceed, on the other hand, decomposition of monomeric silane coupling agent does not occur, as shown in FIG. 2, the weight loss in thermogravimetric analysis is mostly the weight loss at 500°C or higher resulting from decomposition of condensate of dimer or more, as a result of which the hydrolysis and dehydration (condensation) reactions for the filler after surface treatment no longer proceed due to moisture in the air and the like, allowing the filler to exhibit excellent storage stability. In addition thereto, the silane coupling agent has a structure of silanol groups being sufficiently cross-linked, so that the dental composition fabricated exhibits high mechanical properties. In case of using an organic-inorganic composite filler, the mass fraction of an organic component in the organic-inorganic composite filler is subtracted from the weight loss rate at 550°C in thermogravimetric analysis, to calculate the weight loss rate of the silane coupling agent at 550°C followed by calculation of the ratio of the weight loss rate at 250°C in thermogravimetric analysis to the calculated weight loss rate of the silane coupling agent at 550°C to then enable evaluation of degree of condensation of the silane coupling agent.

[(c2-2) Filler having an average primary particle size of 1 to 100 nm]

Among the (c2) surface-treated fillers, it is preferable to contain the (c2-2) filler having an average primary particle size of 1 to 100 nm in the dental composition of the present invention in order to impart rheological properties. The (c2-2) filler having an average primary particle size of 1 to 100 nm may be surface-treated with an aged surface treatment liquid comprising a silane coupling agent and a solvent. The average particle size of a primary particle refers to the diameter of one particle (primary particle) constituting powder. The average particle size used herein can be an average particle size calculated based on the volume-based particle size distribution as measured by, for example, a laser diffraction particle size distribution measurement apparatus, and can be measured, for example, by a laser diffraction particle size measurement apparatus (Microtrac MT3300EXII: manufactured by Nikkiso Co., Ltd.). In addition thereto, the primary particle size can also be measured by dynamic light scattering particle size measurement, or by using electron microscope photographs for those in which primary particles are firmly aggregated to form a secondary particle. Examples of a method for hydrophobizing the (c2-2) filler include surface treatment with modified silicone oil such as dimethylsilicone oil, and/or surface treatment with a silane coupling agent having an alkylsilyl group which may have one or more selected from a trimethylsilyl group, a dimethylsilyl group, a methylsilyl group, or a (meth)acryloyl group having an alkyl chain of C3 to C18.

A commercially available product of the (c2-2) filler having an average primary particle size of 1 to 100 nm, may be used. Specific examples thereof include one or more selected from those produced and commercially available under the trade name Aerosil manufactured by NIPPON AEROSIL CO., LTD., such as Aerosil R972, Aerosil R974, Aerosil R976, Aerosil R711, Aerosil R7200, Aerosil R976S, Aerosil R202, Aerosil R812, Aerosil R812S, Aerosil R805, Aerosil R8200, Aerosil R104, Aerosil R106, Aerosil RY200, Aerosil RX200, Aerosil RY200S, Aerosil RA200H, Aerosil RA200HS, or Alu C 805.

The dental composition containing the (c2-2) filler having an average primary particle size of 1 to 100 nm exhibits moderate thixotropy, resulting in favorable operability thereof. Since the dental composition of the present invention itself exhibits favorable storage stability, it can be expected to exhibit favorable storage stability in spite of a small amount of filler compounded in the dental composition. In general, a large amount of filler compounded may cause reduction in transparency and deterioration of elasticity and flexibility. The transparency, elasticity and flexibility may be required for a dental coating material, a dental lining material, dental cement, a dental bonding material, a dental adhesive material for fixing a mobile tooth, and a dental manicure material. In order to avoid such cases, when a filler is compounded in the dental composition in less amount, its operability may be impaired. In such a case, the (c2-2) filler having an average primary particle size of 1 to 100 nm, with a small amount thereof which can expect to impart rheological properties and improve operability, can be preferably used. It is preferable to contain the (c2-2) filler having an average primary particle size of 1 to 100 nm in an amount of 1 to 30 parts by mass relative to 100 parts by mass of the (A) polymerizable monomer. The amount less than 1 part by mass may not allow rheological properties to be exhibited, and the amount of 30 parts by mass or more may impair operability of the dental composition.

<Other component>

Also, the dental composition of the present invention may contain a component other than the above-described components (A) to (C) as long as effects of the present invention are not impaired. For example, polymerization inhibitors such as hydroquinone, hydroquinone monomethyl ether, and 2,5-ditertiarybutyl-4-methylphenol; mercaptan compounds such as an α-alkylstyrene compound, n-butyl mercaptan, and n-octyl mercaptan; chain transfer agents such as terpenoid-based compounds such as limonene, myrcene, α-terpinene, β-terpinene, γ-terpinene, terpinolene, β-pinene, and α-pinene; fluorescent agents such as dimethyl-2,5-dihydroxyterephthalate, diethyl-2,5-dihydroxyterephthalate, dimethyl-2,5-diaminoterephthalate, diethyl-2,5-diaminoterephthalate, dimethylaminoterephthalate, diethylaminoterephthalate, 2,5-thiophenediylbis(5-tert-butyl-1,3-benzoxazole), 2,2'-(1,2-ethylenediyldi-4,1-phenylene)bisbenzoxazole, and o-phthalaldehyde; ultraviolet-ray absorbers such as 2-(2H-benzotriazol-2-yl)-p-cresol, 2-(2-hydroxy-5-tert-octylphenyl)-benzotriazole, 2-[2-hydroxy-5-[2-(methacryloyloxy)-ethyl]phenyl]-2H-benzotriazole, 2-(5-chloro-2-benzotriazolyl)-6-tert-butyl-p-cresol, 2-hydroxy-4-methoxybenzophenone, 2,2'-dihydroxy-4,4'-dimethoxybenzophenone, 2-hydroxy-4-n-octyloxybenzophenone, and 2-(4,6-diphenyl-1,3,5-triazin-2-yl)-5-[2-(2-ethylhexanoyloxy)ethoxy]phenol, metal scavengers such as an aminocarboxylic acid-based chelating agent and a phosphonic acid-based chelating agent, a discoloration inhibitor, an antibacterial agent, a coloring pigment, water and a solvent that can be mixed therewith in an arbitrary ratio, and other conventionally publicly known additives, can be arbitrarily added, if necessary.

A method for producing the dental composition of the present invention is not limited. An example of a general method for producing the dental composition includes a method for fabricating a matrix obtained by preliminarily mixing the (A) polymerizable monomer and the (B) polymerization initiator by a known method such as a planetary centrifugal mixer, a tumbler mixer, a mix rotor, a dissolver, or a planetary mixer, then kneading the matrix and the (C) filler by a known method such as a planetary centrifugal mixer, a tumbler mixer, a mix rotor, a dissolver, or a planetary mixer, and removing air bubbles under reduced pressure to prepare uniform paste. In the present invention as well, a dental composition can be

produced without any problems by the above-described production method.

In the present invention, a pasty dental composition may be prepared by fabricating a matrix containing a polymerizable monomer and a polymerization initiator and mixing the matrix with a filler. The fabrication of matrix in such a manner enables the polymerization initiator to be dissolved in the matrix, thereby making it possible to homogeneously disperse the polymerization initiator in the paste. In particular, in case of not having dissolved a polymerization initiator that is solid at room temperature in the matrix, sedimentation and variations in performance result. Upon fabrication of matrix, the filler may be partially mixed, if necessary. Also, in fabricating paste, with a premise that the filler is homogeneously dispersed without sedimentation thereof, the filler having an average primary particle size of 100 nm or less may be compounded and then mixed with a filler having a size exceeding 100 nm.

The dental composition of the present invention is preferably used in a dental adhesive, a dental composite resin, a dental core construction material, dental resin cement, a dental coating material, a dental pit and fissure sealant, a dental manicure material, a dental adhesive for fixing a mobile tooth, a dental cutting material, and a material for dental 3D printers, and is particularly preferably used in the dental composite resin.

A consistency retention ratio of dental compositions, in which each of the (c2-1) filler having an average primary particle size of greater than 100 nm, surface-treated with a silane coupling agent after storage at 50°C for 90 days after surface treatment and the (c2-1) filler having an average primary particle size of greater than 100 nm, surface-treated with a silane coupling agent immediately after surface treatment, has compounded in the above-described compositions, is preferably 85% or more, more preferably 90% or more, and particularly preferably 95% or more. The consistency retention ratio is calculated by (([consistency of the dental composition compounded with the (c2-1) filler having an average primary particle size of greater than 100 nm, surface-treated with a silane coupling agent after storage at 50°C for 90 days after surface treatment]/[consistency of the dental composition compounded with the (c2-1) filler having an average primary particle size of greater than 100 nm, surface-treated with a silane coupling agent immediately after surface treatment]) × 100).

An increased ratio in extrusion load of the dental compositions, in which each of the (c2-1) filler having an average primary particle size of greater than 100 nm, surface-treated with a silane coupling agent after storage at 50°C for 90 days after surface treatment and the (c2-1) filler having an average primary particle size of greater than 100 nm, surface-treated with a silane coupling agent immediately after surface treatment, has been compounded in the above-described dental compositions, is preferably 10% or less, more preferably 7% or less, and particularly preferably 5% or less. The increased ratio in extrusion load is calculated by (([dental composition compounded with the (c2-1) filler having an average primary particle size of greater than 100 nm, surface-treated with a silane coupling agent after storage at 50°C for 90 days after surface treatment]/[dental composition compounded with the (c2-1) filler having an average primary particle size of greater than 100 nm, surface-treated with a silane coupling agent immediately after surface treatment]) × 100).

## Examples

The present invention will be described in more detail and specifically below by way of Examples, but the present invention is not limited to these Examples.

The materials and their abbreviations used in Examples and Comparative Examples are shown below.

[(A) Polymerizable monomer]

- Bis-GMA: 2,2-Bis[4-(3-methacryloyloxy-2-hydroxypropoxy)phenyl]propane
- D-2.6E: 2,2-Bis(4-methacryloyloxypolyethoxyphenyl)propane (average number of ethyleneoxy groups added: 2.6)
- D-4E: 2,2-Bis(4-methacryloyloxypolyethoxyphenyl)propane (average number of ethyleneoxy groups added: 4)
- 3G: Triethylene glycol dimethacrylate

[(B) Polymerization initiator]

[(B-1) Photosensitizer]

- CQ: Camphorquinone

[(B-2) Photoacid generator]

- DPI: Diphenyliodonium hexafluorophosphate

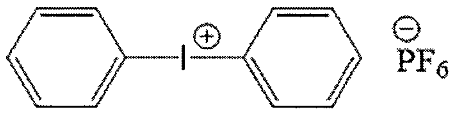

- IFP: p-Cumenyl (p-tolyl) iodonium tris(pentafluoroethyl) trifluorophosphate

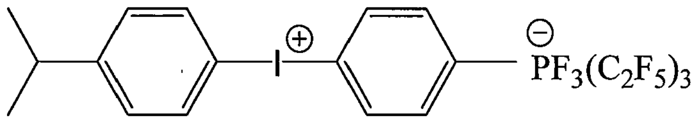

- IFB: Bis(4-tert-butylphenyl) iodonium tetrakis (pentafluorophenyl) borate

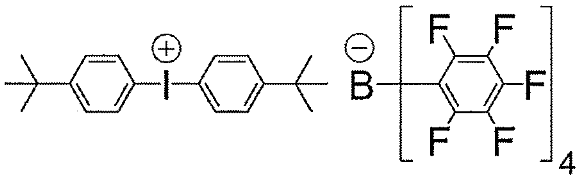

- IFG: Bis(4-tert-butylphenyl) iodonium (tetrakispentafluorophenyl) gallate

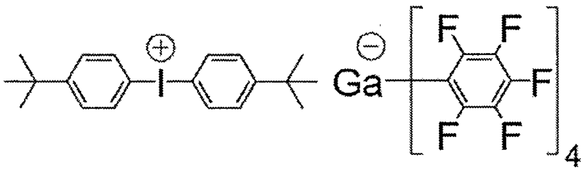

[(B-3) Polymerization accelerator]

- TBA: Tribenzylamine
- DM: N,N-Dimethylaminoethyl methacrylate
- DMBE: N,N-Dimethylaminobenzoic acid ethyl ester

[(c2-1) Filler]

A method for producing each (c2-1) filler used in preparation of dental composition will be described below.

(Production Example 1: Production of c2-1-1 filler)

Various raw materials such as silicon dioxide, aluminum oxide, boron oxide, sodium fluoride, and strontium carbonate were mixed, and the mixture was then melted at 1400°C to obtain A glass (glass composition: $SiO_2$: 22.5% by mass, $Al_2O_3$: 20.0% by mass, $B_2O_3$: 12.3% by mass, SrO: 35.7% by mass, $Na_2O$: 2.5% by mass, and F: 7.0% by mass). The obtained A glass was then pulverized using a vibration mill, and further pulverized using a wet bead mill. To 100 g of the obtained pulverized material was added 4.5 g of a low condensate of a silane compound "MKC Silicate MS56S" ($SiO_2$ content 56.0% by mass, degree of polymerization 2 to 100, manufactured by Mitsubishi Chemical Corporation), and the mixture was stirred and mixed for approximately 90 minutes. After having mixed for a prescribed time, the resulting treated slurry was aged at 50°C for 40 hours in a hot air dryer, then raised to a temperature of 150°C and held for 6 hours, and then cooled to obtain a heat-treated product. The resulting heat-treated product was placed in a Henschel mixer and crushed at

1,800 rpm for 5 minutes to obtain an (1) polysiloxane-treated product (the "(1) polysiloxane-treated product" is also referred to as "NF1") having favorable flowability after the crushing. The 50% average particle size of this (1) polysiloxane-treated product as measured using a laser diffraction particle size measurement apparatus (Microtrack SPA: manufactured by Nikkiso Co., Ltd.), was 1.0 μm.

(Silane treatment)

A surface treatment liquid was prepared by mixing 10.0 g of 8-methacryloyloxyoctyltrimethoxysilane, 0.5 g of a phosphoric acid aqueous solution having a pH of 1.9, and 5.0 g of ethanol into a sealed container, and the surface treatment liquid was aged in a thermostatic chamber at 70°C for 48 hours, and then mixed with 100.0 g of NF1 (average particle size 1.0 μm) under stirring in a Henschel mixer for 30 minutes. Thereafter, the mixture was heat-treated at 100°C for 15 hours to obtain a c2-1-1 filler. The obtained c2-1-1 filler was subjected to thermogravimetric analysis under a nitrogen atmosphere at a heating rate of 10°C/min. As a result, the weight loss rate at 250°C was 21% of the weight loss rate at 550°C, was 21%.

(Production Examples 2 to 40: Production of c2-1-2 to c2-1-40 fillers)

The c2-1-2 to c2-1-40 fillers were obtained in the similar manner as in Production Example 1, except that the compositions, conditions, and the like were changed as shown in Tables 1 and 2.

[Table 1]

| | C2-1- | | | | | | | | | | | | | | | | | | | |
|---|---|---|---|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 | 17 | 18 | 19 | 20 |
| NF1 | O | O | O | O | O | O | O | O | O | O | O | O | O | O | O | O | O | O | O | |
| NF2 | | | | | | | | | | | | | | | | | | | | O |
| NF3 | | | | | | | | | | | | | | | | | | | | |
| NF4 | | | | | | | | | | | | | | | | | | | | |
| SiC1 | O | O | O | O | O | O | O | O | O | O | O | O | O | O | O | O | O | O | O | |
| SiC2 | | | | | | | | | | | | | | | | | | | | O |
| SiC3 | | | | | | | | | | | | | | | | | | | | |
| SiC4 | | | | | | | | | | | | | | | | | | | | |
| SiC5 | | | | | | | | | | | | | | | | | | | | |
| PA | O | O | O | O | | | | | | | O | O | O | | | | O | | | O |
| AA | | | | | O | O | O | | | | | | | | | | | O | | |
| WT | | | | | | | | O | O | O | | | | O | O | O | | | O | |
| EtOH | O | O | O | O | O | | O | O | O | O | | O | O | O | O | O | O | O | | O |
| Acetone | | | | | | O | | | | | O | | | | | | | | O | |
| AT (°C) | 70 | 70 | 70 | 70 | 70 | 50 | 70 | 70 | 70 | 70 | 23 | 23 | 23 | 23 | 23 | 23 | - | - | - | 23 |
| AH (h) | 48 | 24 | 8 | 2 | 24 | 8 | 2 | 24 | 8 | 2 | 24 | 8 | 2 | 24 | 8 | 2 | 0 | 0 | 0 | 24 |
| DR (%) | 21 | 22 | 22 | 25 | 25 | 28 | 34 | 33 | 38 | 41 | 23 | 25 | 43 | 61 | 62 | 65 | 66 | 66 | 66 | 23 |

[Table 2]

| | C2-1- | | | | | | | | | | | | | | | | | | | |
|---|---|---|---|---|---|---|---|---|---|---|---|---|---|---|---|---|---|---|---|---|
| | 21 | 22 | 23 | 24 | 25 | 26 | 27 | 28 | 29 | 30 | 31 | 32 | 33 | 34 | 35 | 36 | 37 | 38 | 39 | 40 |
| NF1 | | | | | | | | | | | | | | | | | | | | |
| NF2 | O | O | O | O | O | O | O | O | O | | | | | | | | | | | |
| NF3 | | | | | | | | | | O | O | O | O | O | O | O | O | O | | |
| NF4 | | | | | | | | | | | | | | | | | | | O | O |
| SiC1 | | | | | | | | | | | | | | | | | | | O | O |
| SiC2 | O | O | O | O | O | | | | | | | | | | | | | | | |
| SiC3 | | | | | | O | O | O | O | | | | | | | | | | | |
| SiC4 | | | | | | | | | | O | O | O | O | | | | | | | |
| SiC5 | | | | | | | | | | | | | | O | O | O | O | O | | |
| PA | O | O | O | | | O | | | O | O | | | | O | O | | | | O | O |
| AA | | | | O | | | O | | | | O | | O | | | O | | | | |
| WT | | | | | O | | | O | | | | O | | | | | O | O | | |
| EtOH | | O | O | O | O | O | O | | O | O | | O | O | O | O | | O | O | O | O |
| Acetone | O | | | | | | | O | | | O | | | | | O | | | | |
| AT (℃) | 23 | 23 | 23 | 70 | 23 | 70 | 23 | 50 | - | 23 | 50 | 23 | - | 70 | 70 | 23 | 70 | - | 70 | - |
| AH (h) | 8 | 6 | 2 | 8 | 2 | 8 | 2 | 24 | 0 | 2 | 24 | 8 | 0 | 24 | 12 | 8 | 2 | 0 | 24 | 0 |
| DR (%) | 22 | 23 | 22 | 22 | 64 | 24 | 38 | 34 | 66 | 44 | 28 | 65 | 64 | 20 | 22 | 24 | 46 | 65 | 20 | 64 |

The meanings of the terms used in Tables 1 and 2 are as follows.

It is to be noted that the circle (O) in the column for each component in the tables indicates that the same amount of filler, silane coupling agent, catalyst (or catalyst substitute), and solvent were used as in the production of the c2-1-1 filler.

(Filler before surface treatment with a silane coupling agent)

NF1: (1) Polysiloxane-treated product (average particle size 1.0 μm)
NF2: Fluoroaluminoborosilicate glass (average particle size 3.0 μm)
NF3: Polysiloxane-polyacrylic acid-treated product (average particle size 0.5 μm) (see its production method in the following paragraph)
NF4: (2) Polysiloxane-treated product (average particle size 3.0 μm) (see its production method in the following paragraph)

(Silane coupling agent)

SiC1: 8-Methacryloyloxyoctyltrimethoxysilane
SiC2: 3-Methacryloyloxypropyltrimethoxysilane
SiC3: 3-Methacryloyloxypropyltriethoxysilane
SiC4: 4,4-Diethoxy-17-oxo-3,16-dioxa-18-aza-4-silaicosane-20-yl methacrylate
SiC5: 4,4-Diethoxy-17-oxo-3,16,21-trioxa-18-aza-4-silatricosane-23-yl methacrylate

(Catalyst)

PA: Phosphoric acid aqueous solution having a pH of 1.9
AA: Acetic acid aqueous solution having a pH of 3.8
WT: Distilled water (used as a substitute for a catalyst because it does not work as a catalyst.)

(Solvent)

EtOH: Ethanol
Acetone: Acetone

(Aging conditions)

AT (°C): Temperature during aging (°C)
AH (h): Time upon aging (hours)

It is to noted that AT (°C) denoted by the sign "-" and AT (h) denoted by the sign "0" mean that no aging was performed.

DR (%): The weight loss rate at 250°C with respect to the weight loss rate at 550°C obtained as a result of thermogravimetric analysis under a nitrogen atmosphere at a heating rate of 10°C/min

(Production of NF3)

Various raw materials such as silicon dioxide, aluminum oxide, boron oxide, sodium fluoride, and strontium carbonate were mixed, and the mixture was then melted at 1400°C to obtain A glass (glass composition: $SiO_2$: 22.5% by mass, $Al_2O_3$: 20.0% by mass, $B_2O_3$: 12.3% by mass, SrO: 35.7% by mass, $Na_2O$: 2.5% by mass, and F: 7.0% by mass). The obtained A glass was then pulverized using a vibration mill, and further pulverized using a wet bead mill. To 100 g of the obtained pulverized material was added 4.5 g of a low condensate of a silane compound "MKC Silicate MS56S" ($SiO_2$ content 56.0% by mass, degree of polymerization 2 to 100, manufactured by Mitsubishi Chemical Corporation), and the mixture was stirred and mixed for approximately 90 minutes. After having mixed for a prescribed time, the resulting treated slurry was aged at 50°C for 40 hours in a hot air dryer, then raised to a temperature of 150°C and held for 6 hours, and then cooled to obtain a heat-treated product. The resulting heat-treated product was placed in a Henschel mixer and crushed at 1,800 rpm for 5 minutes. After crushing, a polysiloxane-treated product having favorable flowability was obtained. One hundred grams of the resulting heat-treated product was fed in a Henschel mixer, and while it was stirred, 16.0 g of a polyacrylic acid aqueous solution (polymer concentration 13% by mass and weight-average molecular weight 20,000, manufactured by NAKARAI TESQUE, INC.) was sprayed from above of the heat-treated product. After spraying, the powder removed from the mixer was heated at 100°C for 3 hours in a hot air dryer to obtain a polysiloxane-polyacrylic acid-treated product (the "polysiloxane-polyacrylic acid-treated product" is also referred to as "NF3"). The 50% average particle size of this polysiloxane-polyacrylic acid-treated product as measured using a laser diffraction particle size measurement apparatus (Microtrack SPA: manufactured by Nikkiso Co., Ltd.), was 0.5 $\mu$m.

(Production of NF4)

Various raw materials such as silicon dioxide, aluminum oxide, boron oxide, sodium fluoride, and strontium carbonate were mixed, and the mixture was then melted at 1400°C to obtain A glass (glass composition: $SiO_2$: 22.5% by mass, $Al_2O_3$: 20.0% by mass, $B_2O_3$: 12.3% by mass, SrO: 35.7% by mass, $Na_2O$: 2.5% by mass, and F: 7.0% by mass). The obtained A glass was then pulverized using a vibration mill, and further pulverized using a wet bead mill. To 100 g of the obtained pulverized material was added 4.5 g of a low condensate of a silane compound "MKC Silicate MS56S" ($SiO_2$ content 56.0% by mass, degree of polymerization 2 to 100, manufactured by Mitsubishi Chemical Corporation), and the mixture was stirred and mixed for approximately 90 minutes. After having mixed for a prescribed time, the resulting treated slurry was aged at 50°C for 40 hours in a hot air dryer, then raised to a temperature of 150°C and held for 6 hours, and then cooled to obtain a heat-treated product. The resulting heat-treated product was placed in a Henschel mixer and crushed at 1,800 rpm for 5 minutes to obtain a (2) polysiloxane-treated product (the "(2) polysiloxane-treated product" is also referred to as "NF4") having favorable flowability after the crushing. The 50% average particle size of this (2) polysiloxane-product as measured using a laser diffraction particle size measurement apparatus (Microtrack SPA: manufactured by Nikkiso Co., Ltd.), was 3.0 $\mu$m.

[c2-2-1 filler]

Aerosil R8200 (manufactured by Evonik Industries AG)

[c2-2-2 Filler]

A surface treatment liquid was fabricated by mixing 10.0 g of 8-methacryloyloxyoctyltrimethoxysilane, 0.5 g of a phosphoric acid aqueous solution having a pH of 1.9, and 5.0 g of ethanol in a sealed container, and the surface treatment liquid was aged in a thermostatic chamber at 70°C for 24 hours, and then mixed with 100.0 g of Aerosil OX-50 (manufactured by Evonik Industries AG) (primary particle size 40 nm) under stirring in a Henschel mixer for 30 minutes.

Thereafter, the mixture was heat-treated at 100°C for 15 hours to obtain a c2-2-2 filler. The obtained c2-2-2 filler was subjected to thermogravimetric analysis under a nitrogen atmosphere at a heating rate of 10°C/min. As a result, the weight loss rate at 250°C was 15% of the weight loss rate at 550°C.

[c2-2-3 Filler]

A surface treatment liquid was fabricated by mixing 10.0 g of 8-methacryloyloxyoctyltrimethoxysilane, 0.5 g of a phosphoric acid aqueous solution having a pH of 1.9, and 5.0 g of ethanol in a sealed container, and the surface treatment liquid was aged in a thermostatic chamber at 70°C for 24 hours, and then mixed with 100.0 g of Aerosil 200 (manufactured by Evonik Industries AG) (primary particle size 12 nm) under stirring in a Henschel mixer for 30 minutes. Thereafter, the mixture was heat-treated at 100°C for 15 hours to obtain a c2-2-3 filler. The obtained c2-2-3 filler was subjected to thermogravimetric analysis under a nitrogen atmosphere at a heating rate of 10°C/min. As a result, the weight loss rate at 250°C was 14% of the weight loss rate at 550°C.

[Ultraviolet-ray absorber]

- HOBP: 2-Hydroxy-4-n-octyloxybenzophenone [Polymerization inhibitor]
- MeHQ: p-Methoxyphenol [Fluorescent agent]
- DHT: Diethyl-2,5-dihydroxyterephthalate

<Production method of dental composition of Example 1>

A wide-mouthed plastic container was charged with 60 parts by weight of Bis-GMA, 40 parts by weight of 3G, 0.1 parts by weight of CQ, 3 parts by weight of IFB, 3 parts by mass of TBA, 0.01 parts by mass of MeHQ, 0.01 parts by mass of DHT, and 0.01 parts by mass of HOBP, which were mixed for 48 hours at 50°C and 100 rpm using a VMRC-5 mix rotor to fabricate a matrix. The obtained matrix was then kneaded with 183 parts by mass of the c2-1-1 and 3 parts by mass of the c2-2-1 in a planetary mixer and filled into a PP syringe to prepare a dental composition. It is to be noted that in Tables 3 and 4, the abbreviation for each component are followed by parts by mass of each component in each parenthesis.

<Production method of dental composition in other Examples and Comparative Examples>

Each dental composition was prepared in the same manner as in Example 1, except that the component and the content were changed as shown in Tables 3 and 4.

[Table 3]

| | Polymerizable monomer (A) | Photoacid generator (B-2) | Polymerization accelerator (B-3) | Filler (C) |
|---|---|---|---|---|
| Example 1 | Bis-GMA (60), 3G (40) | IFB (3) | TBA (3) | c2-1-1 (183), c2-2-1 (3) |
| Example 2 | Bis-GMA (60), 3G (40) | IFB (3) | TBA (3) | c2-1-2 (183), c2-2-1 (3) |
| Example 3 | Bis-GMA (60), 3G (40) | - | DMBE (2) | c2-1-3 (267), c2-2-1 (2) |
| Example 4 | Bis-GMA (60), 3G (40) | IFB (3) | TBA (3) | c2-1-4 (183), c2-2-1 (3) |
| Example 5 | Bis-GMA (57), 3G (40), 6-MHPA (3) | IFB (3) | TBA (3) | c2-1-5 (183), c2-2-1 (3) |
| Example 6 | Bis-GMA (60), 3G (40) | - | DMBE (2) | c2-1-6 (183), c2-2-2 (3) |
| Example 7 | Bis-GMA (60), 3G (40) | - | DMBE (2) | c2-1-7 (183), c2-2-1 (3) |
| Example 8 | Bis-GMA (60), 3G (40) | - | DMBE (2) | c2-1-8 (183), c2-2-1 (3) |

(continued)

| | Polymerizable monomer (A) | Photoacid generator (B-2) | Polymerization accelerator (B-3) | Filler (C) |
|---|---|---|---|---|
| Example 9 | Bis-GMA (60), 3G (40) | IFB (3) | TBA (3) | c2-1-9 (241), c2-2-1 (3) |
| Example 10 | Bis-GMA (60), 3G (40) | IFB (3) | TBA (3) | c2-1-10 (183), c2-2-1 (3) |
| Example 11 | Bis-GMA (57), 3G (40), 6-MHPA (3) | - | DMBE (2) | c2-1-11 (183), c2-2-1 (3) |
| Example 12 | Bis-GMA (60), 3G (40) | IFP (3) | TBA (3) | c2-1-12 (183), c2-2-1 (3) |
| Example 13 | Bis-GMA (60), 3G (40) | - | DMBE (2) | c2-1-13 (183), c2-2-1 (3) |
| Example 14 | Bis-GMA (15), D-2.6E (60), 3G (25) | IFG (3) | TBA (3) | c2-1-20 (183), c2-2-1 (3) |
| Example 15 | Bis-GMA (15), D-2.6E (60), 3G (25) | IFG (3) | TBA (3) | c2-1-21 (183), c2-2-1 (3) |
| Example 16 | Bis-GMA (15), D-2.6E (60), 3G (25) | IFG (3) | TBA (3) | c2-1-22 (183), c2-2-3 (3) |
| Example 17 | Bis-GMA (15), D-2.6E (60), 3G (25) | IFG (3) | TBA (3) | c2-1-23 (111), c2-2-1 (4) |
| Example 18 | Bis-GMA (15), D-2.6E (60), 3G (25) | IFG (3) | TBA (3) | c2-1-24 (241), c2-2-1 (3) |
| Example 19 | Bis-GMA (15), D-2.6E (60), 3G (25) | DPI (0.5) | DM (3) | c2-1-26 (183), c2-2-1 (3) |
| Example 20 | Bis-GMA (15), D-2.6E (60), 3G (25) | DPI (0.5) | DM (3) | c2-1-27 (183), c2-2-1 (3) |

[Table 4]

| | Polymerizable monomer (A) | Photoacid generator (B-2) | Polymerization accelerator (B-3) | Filler (C) |
|---|---|---|---|---|
| Example 21 | Bis-GMA (15), D-2.6E (60), 3G (25) | DPI (0.5) | DM (3) | c2-1-28 (183), c2-2-1 (3) |
| Example 22 | Bis-GMA (10), D-2.6E (45), D-4E (20), 3G (25) | - | DMBE (2) | c2-1-30 (183), c2-2-1 (3) |
| Example 23 | Bis-GMA (10), D-2.6E (45), D-4E (20), 3G (25) | - | DMBE (2) | c2-1-31 (154), c2-2-1 (3) |
| Example 23 | Bis-GMA (10), D-2.6E (45), D-4E (20), 3G (25) | - | DMBE (2) | c2-1-34 (241), c2-2-1 (3) |
| Example 24 | Bis-GMA (10), D-2.6E (45), D-4E (20), 3G (25) | - | DMBE (2) | c2-1-35 (183), c2-2-1 (3) |
| Example 25 | Bis-GMA (10), D-2.6E (45), D-4E (20), 3G (25) | - | DMBE (2) | c2-1-36 (183), c2-2-1 (3) |
| Example 26 | Bis-GMA (10), D-2.6E (45), D-4E (20), 3G (25) | - | DMBE (2) | c2-1-37 (183), c2-2-1 (3) |
| Example 27 | Bis-GMA (10), D-2.6E (45), D-4E (20), 3G (25) | IFB (3) | TBA (3) | c2-1-2 (110), c2-1-39 (203), c2-2-1 (2) |
| Example 28 | Bis-GMA (10), D-2.6E (45), D-4E (20), 3G (25) | IFB (3) | TBA (3) | c2-1-2 (147), c2-1-39 (274), c2-2-1 (2) |
| Comparative Example 1 | Bis-GMA (60), 3G (40) | IFP (3) | TBA (3) | c2-1-14 (183), c2-2-1 (3) |

(continued)

| | Polymerizable monomer (A) | Photoacid generator (B-2) | Polymerization accelerator (B-3) | Filler (C) |
|---|---|---|---|---|
| **Comparative Example 2** | **Bis-GMA (60), 3G (40)** | **-** | **DMBE (2)** | **c2-1-15 (183), c2-2-1 (3)** |
| **Comparative Example 3** | **Bis-GMA (60), 3G (40)** | **IFP (3)** | **TBA (3)** | **c2-1-16 (241), c2-2-1 (3)** |
| **Comparative Example 4** | **Bis-GMA (60), 3G (40)** | **IFP (3)** | **TBA (3)** | **c2-1-17 (267), c2-2-1 (3)** |
| **Comparative Example 5** | **Bis-GMA (60), 3G (40)** | **IFP (3)** | **TBA (3)** | **c2-1-18 (183), c2-2-1 (3)** |
| **Comparative Example 6** | **Bis-GMA (60), 3G (40)** | **-** | **DMBE (2)** | **c2-1-19 (183), c2-2-1 (3)** |
| **Comparative Example 7** | **Bis-GMA (15), D-2.6E (60), 3G (25)** | **IFG (3)** | **TBA (3)** | **c2-1-25 (183), c2-2-1 (3)** |
| **Comparative Example 8** | **Bis-GMA (15), D-2.6E (60), 3G (25)** | **DPI (0.5)** | **DM (3)** | **c2-1-29 (183), c2-2-1 (3)** |
| **Comparative Example 9** | **Bis-GMA (10), D-2.6E (45), D-4E (20), 3G (25)** | **-** | **DMBE (2)** | **c2-1-32 (183), c2-2-1 (3)** |
| **Comparative Example 10** | **Bis-GMA (10), D-2.6E (45), D-4E (20), 3G (25)** | **-** | **DMBE (2)** | **c2-1-33 (183), c2-2-1 (3)** |
| **Comparative Example 11** | **Bis-GMA (10), D-2.6E (45), D-4E (20), 3G (25)** | **-** | **DMBE (2)** | **c2-1-38 (183), c2-2-1 (3)** |
| **Comparative Example 12** | **Bis-GMA (10), D-2.6E (45), D-4E (20), 3G (25)** | **IFB (3)** | **TBA (3)** | **c2-1-17 (110), c2-1-40 (203), c2-2-1 (2)** |

Test methods used in Examples and Comparative Examples are as follows.

(1) Initial consistency

The prepared dental composition was left to stand for one day in a thermostatic chamber at 25°C (humidity 50%), and then 300 $mm^3$ of the dental composition was weighed out on a glass plate. A glass plate was placed on top of the dental composition, and a weight of 385 g was further placed on top of the glass plate, and then the composition was left to stand for three minutes. With an elapsed time of three minutes, the weight was removed, a dimension between parallel tangents of the dental composition, which had spread out in a circle was measured at two locations, and an average value of the dimensions at the two locations was taken as the initial consistency (mm).

(2) Consistency after accelerated test

The dental composition prepared using the filler that had been left to stand for 90 days in a thermostatic chamber at 50°C (humidity 50%) was used to measure a consistency in the same manner as in (1) above.

Also, a consistency retention ratio (%) was calculated according to formula ($\alpha$) to evaluate storage stability of the dental composition. It is to be noted that the consistency retention ratio of 85% or more is determined to have favorable storage stability.

Consistency retention ratio (%) = consistency after accelerated test/initial consistency $\times$ 100 Formula ($\alpha$)

(3) Initial extrusion load

The extrusion load was evaluated as an index of extrusion forcefulness upon discharge of the composition from a syringe. For measurement of extrusion load, a syringe made of a polyolefin resin (container for Beautifil Flow Plus X) and a needle tip (20G, inner diameter of a needle portion 0.68 mm) attached to a tip side of the syringe were used. A storage container is configured of a material that is not transparent to environmental light.

The prepared dental composition (paste) was degassed under vacuum, 2.0 ml thereof was filled into a syringe, a needle tip was attached to the tip of the syringe, and the paste was discharged from the tip of the needle tip by pressing the

plunger. An extrusion load at this time was measured using an Instron universal testing machine (Instron 5943, manufactured by Instron). The storage container was set vertically, and a crosshead equipped with a jig was allowed to descend under the conditions of 20 mm/min and a distance of 7 mm to apply a load to the paste while being discharged. The maximum load at this time was taken as the extrusion load. The extrusion load was measured at 25°C.

(4) Extrusion load after accelerated test

The dental composition (paste) prepared using a filler that had been left to stand in a thermostatic chamber at 50°C (humidity 50%) for 90 days, was degassed under vacuum, and 2.0 ml of the composition was filled into a syringe, and an extrusion load was measured in the same manner as in (3) above.

Also, an increased ratio in extrusion load (%) was calculated according to formula (β) to evaluate storage stability of the dental composition. It is to be noted that an increased ratio in extrusion load of 10% or less, is determined to have favorable storage stability.

Increased ratio in extrusion load (%) = extrusion load after accelerated test/initial extrusion load × 100 Formula (β)

(5) Bending strength

The dental composition prepared was filled into a stainless steel mold, and two pieces of cover glass were placed on both sides of the mold, and the dental composition was pressed against the glass plates, and was then irradiated with light for 10 seconds at five locations using a photopolymerization irradiator (PEN Bright, manufactured by Matsukaze Co., Ltd.) to cure the composition. After curing, the cured product was removed from the mold, and the back side was irradiated with light in the same manner to produce a test specimen (25 × 2 × 2 mm: rectangular parallelepiped). The test specimen was immersed in water at 37°C for 24 hours and then subjected to a bending test. The bending test was performed using an Instron universal testing machine (manufactured by Instron) with a distance between supporting points of 20 mm and a crosshead speed of 1 mm/min.

The results of each test shown in Tables 5 and 6 will be described below.

[Table 5]

| Example | Initial consistency (mm) | Consistency after accelerated test (mm) | Consistency retention ratio (%) | Initial extrusion load (kgf) | Extrusion load after accelerated test (kgf) | Increased ratio in extrusion load (%) | Bending strength (MPa) |
|---|---|---|---|---|---|---|---|
| Example 1 | 46.5 | 46.0 | 98.9 | 8.9 | 9.1 | 2.2 | 135 |
| Example 2 | 47.0 | 46.0 | 97.9 | 8.8 | 9.1 | 3.4 | 135 |
| Example 3 | 42.0 | 41.0 | 97.6 | 10.0 | 10.4 | 4.0 | 125 |
| Example 4 | 47.0 | 44.5 | 94.7 | 8.8 | 9.4 | 6.8 | 130 |
| Example 5 | 46.5 | 45.0 | 96.8 | 8.6 | 8.9 | 3.5 | 132 |
| Example 6 | 47.5 | 46.0 | 96.8 | 8.2 | 8.4 | 2.4 | 124 |
| Example 7 | 47.0 | 44.0 | 93.6 | 8.8 | 9.4 | 6.8 | 123 |
| Example 8 | 46.5 | 42.0 | 90.3 | 8.9 | 9.5 | 6.7 | 119 |
| Example 9 | 41.5 | 38.0 | 91.6 | 9.7 | 10.3 | 6.2 | 123 |
| Example 10 | 46.0 | 39.5 | 85.9 | 8.7 | 9.5 | 9.2 | 120 |

(continued)

| Example | Initial consistency (mm) | Consistency after accelerated test (mm) | Consistency retention ratio (%) | Initial extrusion load (kgf) | Extrusion load after accelerated test (kgf) | Increased ratio in extrusion load (%) | Bending strength (MPa) |
|---|---|---|---|---|---|---|---|
| Example 11 | 46.5 | 45.0 | 96.8 | 8.6 | 9.0 | 4.7 | 124 |
| Example 12 | 47.0 | 44.0 | 93.6 | 8.8 | 9.4 | 6.8 | 132 |
| Example 13 | 46.0 | 41.0 | 89.1 | 8.8 | 9.6 | 9.1 | 121 |
| Example 14 | 45.5 | 44.5 | 97.8 | 9.7 | 10.0 | 3.1 | 130 |
| Example 15 | 45.5 | 44.5 | 97.8 | 9.9 | 10.3 | 4.0 | 132 |
| Example 16 | 46.0 | 45.0 | 97.8 | 9.5 | 9.9 | 4.2 | 130 |
| Example 17 | 53.0 | 52.0 | 98.1 | 7.2 | 7.4 | 2.8 | 110 |
| Example 18 | 39.0 | 37.5 | 96.2 | 10.8 | 11.2 | 3.7 | 133 |
| Example 19 | 45.0 | 44.0 | 97.8 | 9.9 | 10.2 | 3.0 | 131 |
| Example 20 | 46.0 | 42.5 | 92.4 | 10.0 | 10.6 | 6.0 | 132 |

[Table 6]

| Example | Initial consistency (mm) | Consistency after accelerated test (mm) | Consistency retention ratio (%) | Initial extrusion load (kgf) | Extrusion load after accelerated test (kgf) | Increased ratio in extrusion load (%) | Bending strength (MPa) |
|---|---|---|---|---|---|---|---|
| Example 21 | 46.0 | 43.0 | 93.5 | 9.7 | 10.3 | 6.2 | 130 |
| Example 22 | 53.0 | 47.5 | 89.6 | 7.9 | 8.6 | 8.9 | 121 |
| Example 23 | 55.0 | 53.5 | 97.3 | 7.4 | 7.7 | 4.1 | 121 |
| Example 23 | 50.0 | 48.5 | 97.0 | 9.0 | 9.4 | 4.4 | 126 |
| Example 24 | 54.5 | 53.0 | 97.2 | 7.6 | 7.8 | 2.6 | 124 |
| Example 25 | 54.5 | 52.5 | 96.3 | 7.6 | 7.9 | 3.9 | 123 |
| Example 26 | 55.5 | 48.0 | 86.5 | 7.4 | 8.1 | 9.5 | 120 |
| Example 27 | 44.0 | 42.5 | 96.6 | 9.2 | 9.5 | 3.3 | 135 |
| Example 28 | 38.0 | 37.0 | 97.4 | 10.7 | 11.1 | 3.7 | 138 |
| Comparative Example 1 | 46.5 | 37.5 | 80.6 | 8.7 | 10.2 | 17.2 | 107 |
| Comparative Example 2 | 46.5 | 37.0 | 79.6 | 8.6 | 10.3 | 19.8 | 101 |
| Comparative Example 3 | 41.0 | 33.0 | 80.5 | 9.8 | 11.5 | 17.3 | 107 |

(continued)

| Example | Initial consistency (mm) | Consistency after accelerated test (mm) | Consistency retention ratio (%) | Initial extrusion load (kgf) | Extrusion load after accelerated test (kgf) | Increased ratio in extrusion load (%) | Bending strength (MPa) |
|---|---|---|---|---|---|---|---|
| Comparative Example 4 | 42.0 | 31.0 | 73.8 | 10.1 | 12.1 | 19.8 | 114 |
| Comparative Example 5 | 46.5 | 34.5 | 74.2 | 8.7 | 10.3 | 18.4 | 114 |
| Comparative Example 6 | 47.0 | 34.0 | 72.3 | 8.9 | 10.5 | 18.0 | 104 |
| Comparative Example 7 | 46.0 | 37.0 | 80.4 | 9.8 | 11.5 | 17.3 | 111 |
| Comparative Example 8 | 45.5 | 36.0 | 79.1 | 9.7 | 11.3 | 16.5 | 114 |
| Comparative Example 9 | 53.0 | 44.0 | 83.0 | 7.9 | 9.4 | 19.0 | 102 |
| Comparative Example 10 | 52.5 | 42.0 | 80.0 | 7.9 | 9.5 | 20.3 | 104 |
| Comparative Example 11 | 54.5 | 44.5 | 81.7 | 7.5 | 9.1 | 21.3 | 101 |
| Comparative Example 12 | 43.5 | 35.0 | 80.5 | 9.2 | 10.8 | 17.4 | 125 |

The compositions of Examples 1 to 28, which used fillers having a weight loss rate at 250°C being 50% or less of the weight loss rate at 550°C upon thermogravimetric analysis under the conditions of a nitrogen atmosphere and a heating rate of 10°C/min, exhibited no decrease in consistency and no increase in extrusion load even after the accelerated test, confirming that they had favorable storage stability.

The compositions of Examples 1 to 28 were also confirmed to have high bending strength. This is believed to be because the hydrolysis and dehydration (condensation) reactions of the silane coupling agent proceeded due to the aging of the surface treatment liquid, resulting in satisfactory formation of cross-linked structure of the silanol groups.

The compositions of Comparative Examples 1 to 12, which used fillers having a weight loss rate at 250°C being more than 50% of the weight loss rate at 550°C upon thermogravimetric analysis under the conditions of a nitrogen atmosphere and a heating rate of 10°C/min, were confirmed to exhibit a decrease in consistency and an increase in extrusion load after the accelerated test.

Industrial Applicability

The present invention provides the dental composition excellent in storage stability and mechanical strength without sacrificing an operational feeling.

## Claims

1. A dental composition, comprising an (A) polymerizable monomer, a (B) polymerization initiator, and a (C) filler, wherein the (C) filler comprises a (c2-1) filler having an average primary particle size of greater than 100 nm, surface-treated with a silane coupling agent, and when the (c2-1) filler having an average primary particle size of greater than 100 nm, surface-treated with a silane coupling agent, undergoes a thermogravimetric analysis under conditions of a heating rate of 10°C/min from 30°C to 550°C in a nitrogen atmosphere, a weight loss rate at 250°C is 50% or less of a weight loss rate at 550°C.

2. The dental composition according to claim 1, wherein the silane coupling agent has a structure represented by formula (1):

[Formula 1]

$$R^3-\underset{\displaystyle R^1_{(3-p)}}{\underset{|}{Si}}-(OR^2)_p$$

wherein $R^1$ and $R^2$ are C1 to C4 alkyl groups optionally having the same or different groups from each other, $R^3$ is a (meth)acryloyl group having a C6 to C15 alkyl group optionally having one or more groups selected from -O-, -S-, -NH-, -C(O)-O-, -O-C(O)-, -O-C(O)-NH-, and -NH-C(O)-O- groups, and
p is 2 or 3.

**3.** The dental composition according to claim 1, wherein the (c2-1) filler having an average primary particle size of greater than 100 nm, surface-treated with a silane coupling agent, is a mixture of a surface treatment liquid comprising the silane coupling agent, a catalyst and a solvent, and a (c1) untreated filler.

**4.** The dental composition according to claim 3, wherein the surface treatment liquid is a mixed solution aged at a temperature below a boiling point of the solvent.

**5.** The dental composition according to claim 1, wherein a consistency retention ratio of a dental composition compounded with the (c2-1) filler having an average primary particle size of greater than 100 nm, surface-treated with a silane coupling agent after storage at 50°C for 90 days after surface treatment, is 85% or more of a consistency of a dental composition compounded with the (c2-1) filler having an average primary particle size of greater than 100 nm, surface-treated with a silane coupling agent immediately after surface treatment.

**6.** The dental composition according to claim 1, wherein an increased ratio in extrusion load of the dental composition compounded with the (c2-1) filler having an average primary particle size of greater than 100 nm, surface-treated with a silane coupling agent after storage at 50°C for 90 days after surface treatment, is 10% or less of an extrusion load of a dental composition compounded with the (c2-1) filler having an average primary particle size of greater than 100 nm, surface-treated with a silane coupling agent immediately after surface treatment.

**7.** The dental composition according to any one of claims 1 to 6, comprising 0.5 to 10 parts by mass of the (B) polymerization initiator and 100 to 900 parts by mass of the (c2-1) filler having an average primary particle size of greater than 100 nm, surface-treated with a silane coupling agent, relative to a total of 100 parts by mass of the (A) polymerizable monomer,
wherein a ratio of content of the (c1) untreated filler to content of a silane coupling agent ([(c1) untreated filler/silane coupling agent], mass ratio, in terms of solid content) in the (c2-1) filler having an average primary particle size of greater than 100 nm, surface-treated with the silane coupling agent that is a mixture of a surface treatment liquid comprising the silane coupling agent, a catalyst and a solvent, and the (c1) untreated filler, is 100/0.5 to 20.

S[FIG. 1]

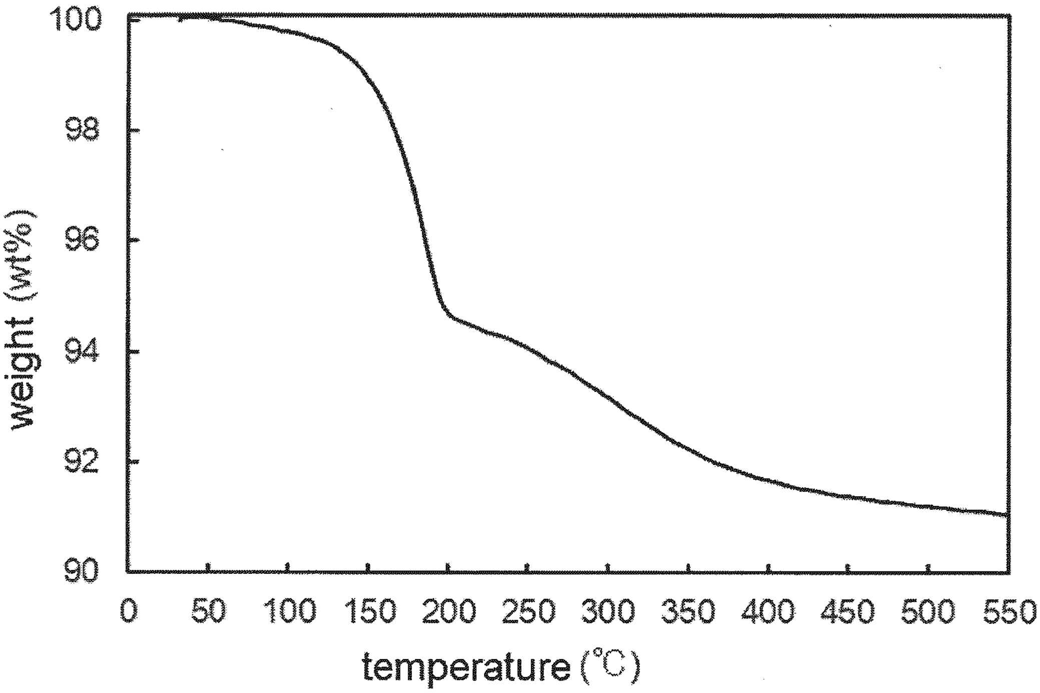
100
98
96
94
92
90
weight (wt%)
0
50
100
150
200
250
300
350
400
450
500
550
temperature (℃)

[FIG. 2]

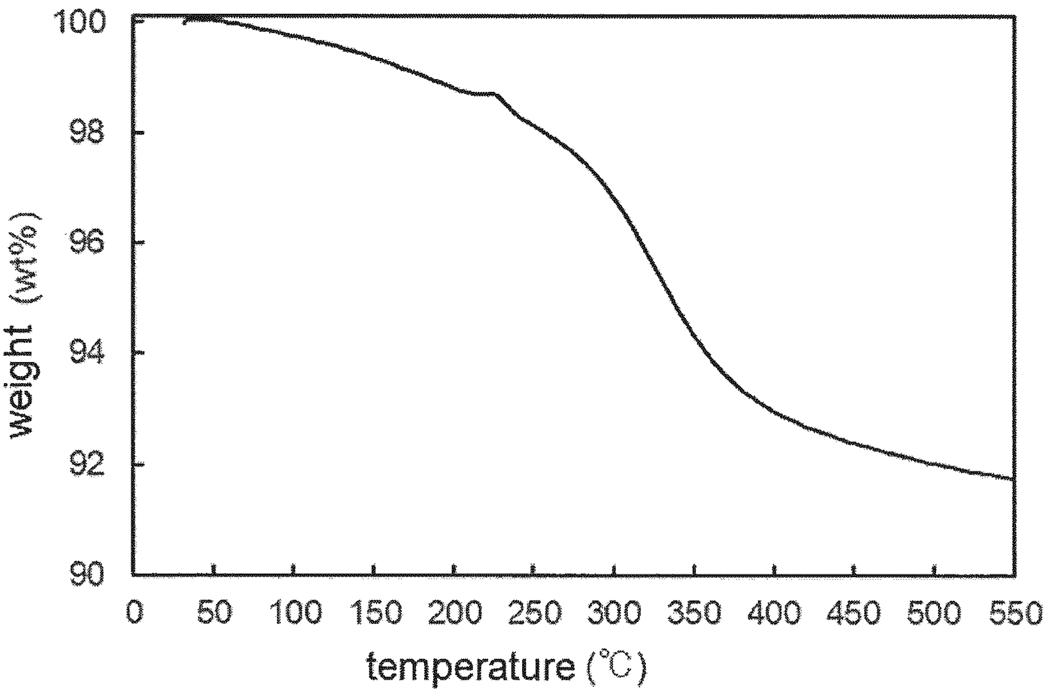
100
98
96
94
92
90
weight (wt%)
0
50
100
150
200
250
300
350
400
450
500
550
temperature (℃)

Europäisches Patentamt
European Patent Office
Office européen des brevets

# EUROPEAN SEARCH REPORT

Application Number
EP 25 20 2411

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/023155 A1 (YAMAMOTO KENZO [JP] ET AL) 27 January 2022 (2022-01-27)<br>* paragraphs [0231] - [0288]; tables 1,5,7,9,10,11,13 *<br>----- | 1-7 | INV.<br>A61K6/17<br>A61K6/62<br>A61K6/76<br>A61K6/887<br>A61K6/20<br>A61K6/30 |
| X | EP 2 982 361 A2 (SHOFU INC [JP])<br>10 February 2016 (2016-02-10)<br>* paragraphs [0187] - [0195]; tables 1-3 *<br>----- | 1-7 | |
| X | US 2022/387264 A1 (MIYATA SHUNSUKE [JP] ET AL) 8 December 2022 (2022-12-08)<br>* paragraphs [0164], [0208] - [0219]; tables 1-3 *<br>----- | 1-7 | |

TECHNICAL FIELDS SEARCHED (IPC)

A61K

The present search report has been drawn up for all claims

2

| Place of search | Date of completion of the search | Examiner |
|---|---|---|
| The Hague | 2 February 2026 | Barenbrug-van Druten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 25 20 2411

This annex lists the patent family members relating to the patent documents cited in the above-mentioned European search report. The members are as contained in the European Patent Office EDP file on
The European Patent Office is in no way liable for these particulars which are merely given for the purpose of information.

02-02-2026

| Patent document cited in search report | Publication date | Patent family member(s) | Publication date |
|---|---|---|---|
| US 2022023155 A1 | 27-01-2022 | CN 113491636 A | 12-10-2021 |
| | | EP 3903759 A1 | 03-11-2021 |
| | | US 2022023155 A1 | 27-01-2022 |
| EP 2982361 A2 | 10-02-2016 | CN 105310891 A | 10-02-2016 |
| | | EP 2982361 A2 | 10-02-2016 |
| | | JP 5716118 B1 | 13-05-2015 |
| | | JP 2016030801 A | 07-03-2016 |
| | | KR 20160014522 A | 11-02-2016 |
| | | KR 20220044261 A | 07-04-2022 |
| | | KR 20230028353 A | 28-02-2023 |
| | | US 2016030298 A1 | 04-02-2016 |
| | | US 2019209439 A1 | 11-07-2019 |
| US 2022387264 A1 | 08-12-2022 | CN 115068346 A | 20-09-2022 |
| | | CN 115068348 A | 20-09-2022 |
| | | EP 4062893 A1 | 28-09-2022 |
| | | EP 4062894 A1 | 28-09-2022 |
| | | EP 4062895 A1 | 28-09-2022 |
| | | US 2022387263 A1 | 08-12-2022 |
| | | US 2022387264 A1 | 08-12-2022 |
| | | US 2022401312 A1 | 22-12-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014083842 A
- WO 2016152659 A
- WO 2020031444 A